# EUROPEAN PATENT APPLICATION

(11) **EP 4 538 314 A1**
(43) Date of publication of application: **16.04.2025**
(21) Application number: 23819688.5
(22) Date of filing: 26.05.2023
(51) Int. Cl.: C08G 81/00, C08G 69/44

(54) **ESTER-AMIDE MULTI-BLOCK COPOLYMER, METHOD FOR PRODUCING ESTER-AMIDE MULTI-BLOCK COPOLYMER, ESTER-AMIDE COPOLYMER, AND METHOD FOR PRODUCING ESTER-AMIDE COPOLYMER**

(30) Priority: 06.06.2022 JP 2022091265; 30.11.2022 JP 2022191365
(71) Applicant: National Institute of Advanced Industrial Science and Technology, Chiyoda-ku Tokyo 100-8921 (JP)
(72) Inventor: TANAKA, Shinji, Tsukuba-shi, Ibaraki 305-8560 (JP); ONO, Hideaki, Tsukuba-shi, Ibaraki 305-8560 (JP); YOSHIDA, Masaru, Tsukuba-shi, Ibaraki 305-8560 (JP); MINAMIKAWA, Hiroyuki, Tsukuba-shi, Ibaraki 305-8560 (JP); ATA, Seisuke, Tsukuba-shi, Ibaraki 305-8560 (JP); KURIHARA, Kazuma, Tsukuba-shi, Ibaraki 305-8560 (JP); KAWAI, Yasutaka, Tsukuba-shi, Ibaraki 305-8560 (JP)
(74) Representative: Groth, Wieland
(86) International application number: PCT/JP2023/019715
(87) International publication number: WO 2023/238706

(57) **Abstract**

An ester-amide multi-block copolymer that is a multi-block copolymer including a polyester-containing block and a polyamide-containing block obtained through ring-opening polymerization of a cyclic lactam, and that is represented by the following formula (1): 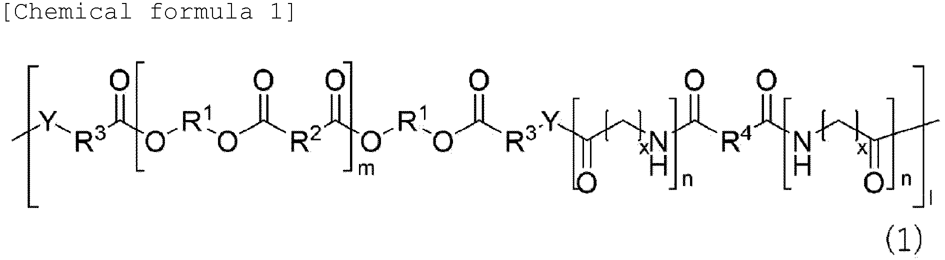 (in the formula (1), R¹ and R² each represent an alkyl chain of 1 to 20 carbon atoms having hydrogen atoms or a substituent, and R³ represents an alkyl chain of 1 to 10 carbon atoms having hydrogen atoms or a substituent; R⁴ represents any one of an aromatic hydrocarbon containing a hetero atom or no hetero atoms, and an aliphatic hydrocarbon chain containing a hetero atom or no hetero atoms; Y is NH, O, or S; m represents an integer of 1 to 60, n represents an integer of 1 to 120, l represents an integer of 2 to 100, and x represents an integer of 1 to 11). A method for producing the above-described multi-block copolymer is also provided.

## Description

### Technical Field

The present invention relates to a novel copolymer and a method for producing the novel copolymer, particularly to an ester-amide block copolymer having a novel copolymer structure and a method for producing the ester-amide block copolymer, and an ester-amide copolymer and a method for producing the ester-amide copolymer.

### Background Art

Having excellent characteristics such as high heat resistance, high mechanical strength, and chemical resistance, a polyamide that has an amide bond is utilized as one of engineering plastics. In particular, polyamides such as nylon 6, nylon 10, and nylon 12 which are obtained through ring-opening polymerization of a cyclic lactam are representative materials and used as thermoplastic engineering plastics and as fiber materials. Nylon 4 has recently attracted attention because it has biodegradability, which other polyamides do not have, while having characteristics such as high heat resistance and high mechanical strength.

Various approaches have been used for modifying the physical properties of such polyamides. For example, PTL 1 discloses, as an example of modification of nylon 4, the production of a polyamide 4 copolymer by performing copolymerization with ε-caprolactam using a basic polymerization catalyst and an initiator having a branched structure during polymerization of 2-pyrrolidone. PTL 2 discloses the production of various block copolymers containing polyamide 4 by introducing an azo group into the macromolecular chain of polyamide 4. PTL 3 discloses the production of an aliphatic polyester amide by using a polyester oligomer having biodegradability as an initiator for polymerization of 2-pyrrolidone. NPL 1 discloses the synthesis of a diblock copolymer of a polybutylene succinate having an ester bond and polyamide 4.

### Citation List

### Patent Literature

PTL 1: Japanese Patent Application Laid-Open No. 2009-155608
PTL 2: Japanese Patent Application Laid-Open No. 2004-331780
PTL 3: Japanese Patent No. 5207343

### Non Patent Literature

NPL 1: Hideaki Ono et al., European Polymer Journal, Volume 163, 2022, 110961

### Summary of Invention

### Technical Problem

In the technologies according to the above-described PTLs 1 to 3 and NPL 1, the production of a block copolymer of a polyamide is described. However, practical application of a block copolymer as a material having excellent moldability is desired.

An object of the present invention is to provide a novel copolymer having excellent moldability and a method for producing the copolymer. In particular, an object of the present invention is to provide a block copolymer having a novel copolymer structure and a method for producing the block copolymer.

### Solution to Problem

The present inventors have found that a composite material having excellent moldability can be obtained by preparing a multi-block copolymer of an aliphatic polyester and a polyamide and have thus completed the present invention. Furthermore, physical properties suitable for a certain application can be obtained by adjusting the blend ratio between the aliphatic polyester and the polyamide.

That is, the present invention includes the following aspects:
An aspect of the present invention relates to (1) an ester-amide multi-block copolymer that is a multi-block copolymer including a polyester-containing block and a polyamide-containing block obtained through ring-opening polymerization of a cyclic lactam, and that is represented by the following formula (1): 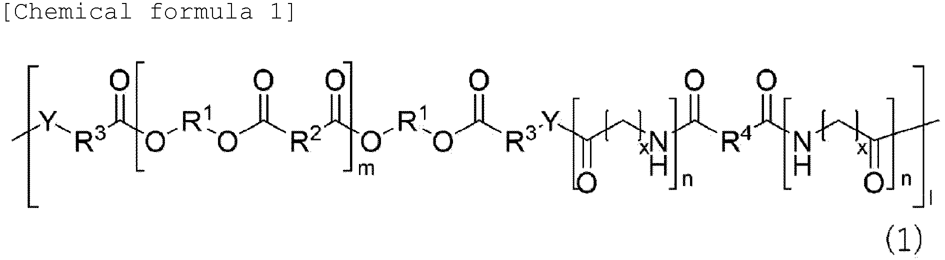 (in the formula (1), R¹ and R² each represent an alkyl chain of 1 to 20 carbon atoms having hydrogen atoms or substituents, and R³ represents an alkyl chain of 1 to 10 carbon atoms having hydrogen atoms or substituents; R¹, R², and R³ may be the same as or different from one another; R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 12 carbon atoms containing no hetero atoms, and an aliphatic hydrocarbon chain of 1 to 10 carbon atoms containing no hetero atoms; Y is NH, O (oxygen), or S (sulfur); m represents an integer of 1 to 60, n represents an integer of 1 to 120, l (alphabet "l") represents an integer of 2 to 100, and x represents an integer of 1 to 11).

In another aspect, the present invention relates to (2) an ester-amide multi-block copolymer that is a multi-block copolymer including a polyester-containing block and a polyamide-containing block obtained through ring-opening polymerization of a cyclic lactam, and that is represented by the following formula (2): 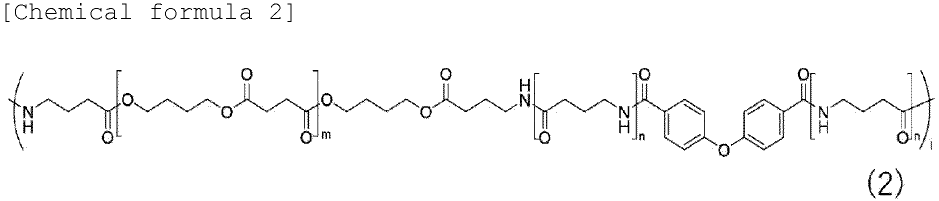 (in the formula (2), m represents an integer of 1 to 60, n represents an integer of 1 to 120, and l represents an integer of 2 to 100).

In yet another aspect, the present invention relates to (3) a film including the ester-amide multi-block copolymer according to (1) or (2) described above.

In yet another aspect, the present invention relates to
(4) a method for producing the ester-amide multi-block copolymer according to (1) described above, including a step of bonding a polyester-containing compound represented by the following formula (3): 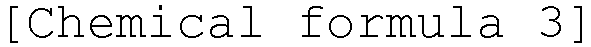 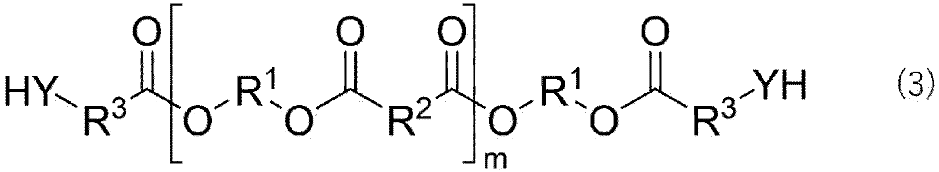 (in the formula (3), R¹ and R² each represent an alkyl chain of 1 to 20 carbon atoms having hydrogen atoms or substituents, and R³ represents an alkyl chain of 1 to 10 carbon atoms having hydrogen atoms or substituents; R¹, R², and R³ may be the same as or different from one another; Y is NH, O (oxygen), or S (sulfur); and m represents an integer of 1 to 60) to
a polyamide-containing compound represented by the following formula (4) obtained through ring-opening polymerization of a cyclic lactam: 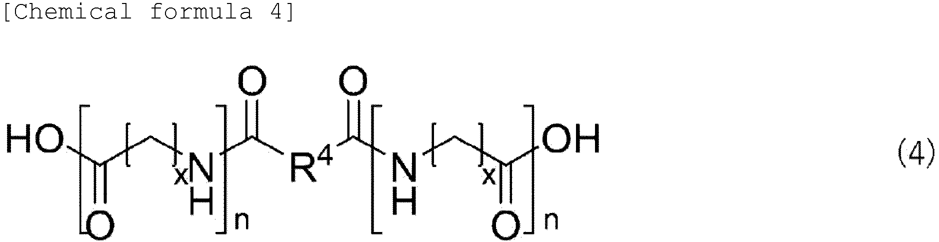 (in the formula (4), R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 12 carbon atoms containing no hetero atoms, and an aliphatic hydrocarbon chain of 1 to 10 carbon atoms containing no hetero atoms; and n represents an integer of 1 to 120, and x represents an integer of 1 to 11)
by forming a bond between a YH group in the compound represented by the above-mentioned formula (3) and a carboxy group in the compound represented by the above-mentioned formula (4).

In yet another aspect, the present invention relates to (5) a method for producing the ester-amide multi-block copolymer according to (2) above, including a step of bonding a polyester-containing compound represented by the following formula (5): 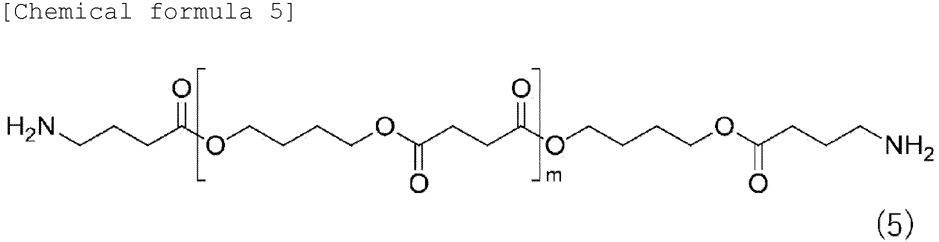 (in the formula (5), m represents an integer of 1 to 60) to
a polyamide-containing compound represented by the following formula (6) obtained through ring-opening polymerization of a cyclic lactam: 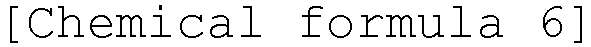 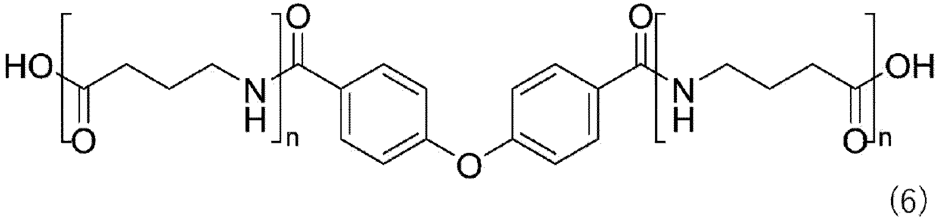 (in the formula (6), n represents an integer of 1 to 120)
by forming a bond between an amino group in the compound represented by the above-mentioned formula (5) and a carboxy group in the compound represented by the above-mentioned formula (6).

Furthermore, in the below-described copolymer of an ester and an amide, it also serves as a composite material having excellent moldability.

In yet another aspect, the present invention relates to (6) an ester-amide copolymer represented by the following formula (19): 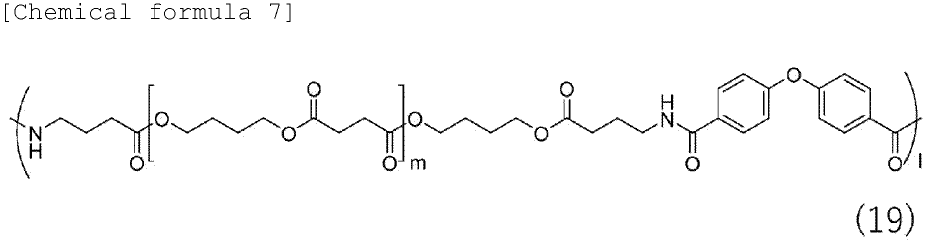 (in the formula (19), m represents an integer of 1 to 60, and l represents an integer of 2 to 100).

In yet another aspect, the present invention relates to (7) a film including the ester-amide copolymer according to (6) described above.

In yet another aspect, the present invention relates to (8) a method for producing the ester-amide copolymer according to (6) described above, including a step of bonding a compound represented by the following formula (5): 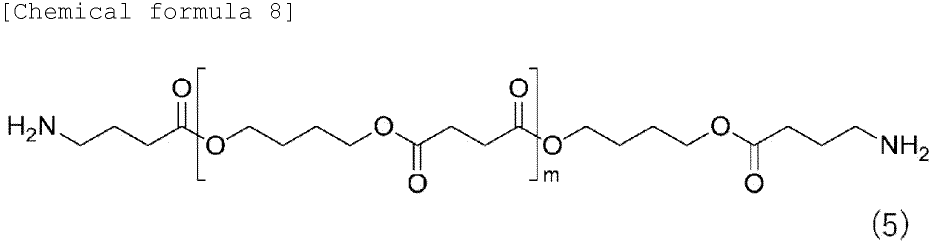 (in the formula (5), m represents an integer of 1 to 60) to
a compound represented by the following formula (20): 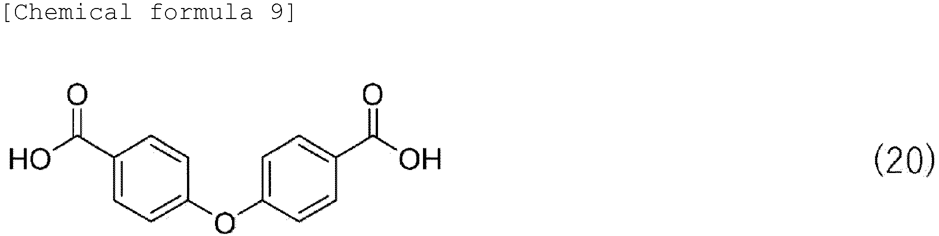
by forming a bond between an amino group in the compound represented by the above-described formula (5) and a carboxy group in the compound represented by the above-described formula (20).

### Advantageous Effects of Invention

According to the present invention, there can be provided a novel copolymer having excellent moldability and a method for producing the copolymer. In particular, there can be provided a block copolymer having a novel copolymer structure and a method for producing the block copolymer.

### Brief Description of Drawings

[Fig. 1] Fig. 1 is a graph showing results of a molecular weight distribution measurement of a both-ends hydroxyl group-terminated PBS (Experiment Example 1).
[Fig. 2] Fig. 2 is a ¹H-NMR spectrum of the both-ends hydroxyl group-terminated PBS (Experiment Example 1).
[Fig. 3] Fig. 3 is a graph showing results of a molecular weight distribution measurement of a both-ends NH-Boc-terminated PBS (Experiment Example 6).
[Fig. 4] Fig. 4 is a ¹H-NMR spectrum of the both-ends NH-Boc-terminated PBS (Experiment Example 6).
[Fig. 5] Fig. 5 is a ¹H-NMR spectrum of a both-ends amino group-terminated PBS (Experiment Example 11).
[Fig. 6] Fig. 6 is a ¹H-NMR spectrum of a PA4 polymerization reaction initiator (Experiment Example 16).
[Fig. 7] Fig. 7 is a graph showing results of a molecular weight distribution measurement of a both-ends carboxy group-terminated PA4 (Experiment Example 17).
[Fig. 8] Fig. 8 is a ¹H-NMR spectrum of the both-ends carboxy group-terminated PA4 (Experiment Example 17).
[Fig. 9] Fig. 9 is a graph showing results of a molecular weight distribution measurement of a PBS-PA4 multi-block copolymer (Experiment Example 23).
[Fig. 10] Fig. 10 is a ¹H-NMR spectrum of the PBS-PA4 multi-block copolymer (Experiment Example 23).
[Fig. 11] Fig. 11 is a graph showing thermal properties of a multi-block copolymer according to an embodiment of the present invention.
[Fig.12] Fig. 12 is a film appearance photograph of a multi-block copolymer according to an embodiment of the present invention.
[Fig.13] Fig. 13 is a graph showing the results of measuring the transmittance of the film of the multi-block copolymer according to the embodiment of the present invention.
[Fig. 14] Fig. 14 is a ¹H-NMR spectrum of a one-end NH-Boc-terminated butanediol (Experiment Example 34).
[Fig. 15] Fig. 15 is a ¹H-NMR spectrum of a both-ends NH-Boc-terminated PBS (Experiment Example 35).
[Fig. 16] Fig. 16 is a ¹H-NMR spectrum of a both-ends amino group-terminated PBS (Experiment Example 36).
[Fig. 17] Fig. 17 is a ¹H-NMR spectrum of an ester-amide copolymer (Experiment Example 37).
[Fig. 18] Fig. 18 is a graph showing thermal properties of an ester-amide copolymer according to another embodiment of the present invention.

### Description of Embodiments

The multi-block copolymer of the present embodiment is a multi-block copolymer including a polyester-containing block and a polyamide-containing block obtained through ring-opening polymerization of a cyclic lactam, and provides a novel ester-amide multi-block copolymer represented by the following formula (1): 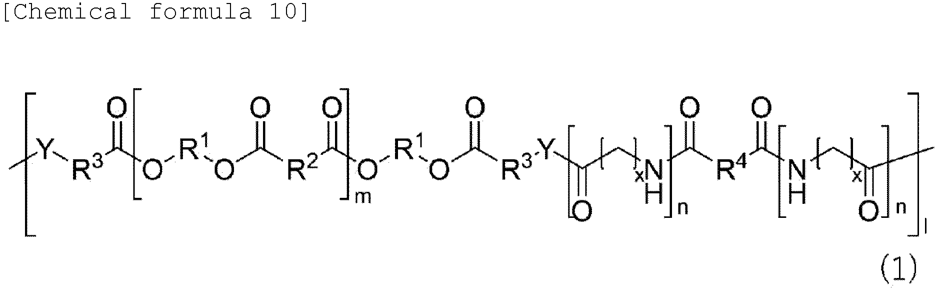 (in the formula (1), R¹ and R² each represent an alkyl chain of 1 to 20 carbon atoms having hydrogen atoms or substituents, and R³ represents an alkyl chain of 1 to 10 carbon atoms having hydrogen atoms or substituents; R¹, R², and R³ may be the same as or different from one another; R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 10 carbon atoms containing no hetero atoms, and an aliphatic hydrocarbon chain of 1 to 10 carbon atoms containing no hetero atoms; Y is NH, O (oxygen), or S (sulfur); m represents an integer of 1 to 60, n represents an integer of 1 to 120, l represents an integer of 2 to 100, and x represents an integer of 1 to 11).

Specifically, the multi-block copolymer of the present embodiment has a structure in which a plurality (l) of units represented by the formula (1) are bonded. Note that, in the present specification, a numerical range includes both its upper and lower limits.

In an embodiment, in the formula (1), x, m, n, and l are any integer selected from the following ranges. x is 1 to 11, preferably 3 to 5 or 11 (an alkyl chain of 3 to 5 or 11 carbon atoms) from the viewpoint of availability of cyclic lactam for use in a reaction step, and more preferably 3 from the viewpoint of biodegradability. m represents an integer of 1 to 60, and, for ease of purification of the product, preferably 10 to 60, and more preferably 15 to 60. n represents an integer of 1 to 120, and, for ease of purification of the product, preferably 5 to 80, and more preferably 5 to 20. l represents an integer of 2 to 100, and, for ease of purification of the product, preferably 2 to 50, and more preferably 2 to 30. The combination of m, n, and l may be any combination of integers such that the number-average molecular weight of the resulting block copolymer becomes 5,000 to 2,000,000, preferably 8,000 to 50,000. When the molecular weight is less than the lower limit, the strength of the resin decreases. When the molecular weight exceeds the upper limit, the solubility of the compound decreases, and a good moldability is not achieved as compared with the case where the molecular weight is equal to or less than the upper limit.

In the formula (1), examples of the "alkyl chain" of R¹ and R² include linear alkyl chains of 1 to 20 carbon atoms. R¹ is preferably an alkyl chain of 2 to 12 carbon atoms, more preferably 2 to 6 carbon atoms, and still more preferably 2 to 4 carbon atoms, from the viewpoint of availability of a divalent alcohol for use in the reaction step. R² is preferably an alkyl chain of 1 to 6 carbon atoms, more preferably 2 to 5 carbon atoms, from the viewpoint of availability of a dicarboxylic acid for use in the reaction step. Examples of the "alkyl chain" of R³ include a linear alkyl chain of 1 to 10 carbon atoms, more preferably 3 to 5 carbon atoms from the viewpoint of availability of a synthetic raw material. In addition, the numbers of carbon atoms of the alkyl chains of R¹, R², and R³ may be the same as or different from one another.

Examples of the substituent that may be contained in R¹, R², and R³ include a hydrocarbon group of 1 to 10 carbon atoms, more preferably 1 to 6 carbon atoms, an oxygen-containing group, a nitrogen-containing group, and a halogen atom.

Specific examples of the hydrocarbon group include an alkyl group, such as a methyl group, an ethyl group, an n-propyl group, an isopropyl group, an n-butyl group, an isobutyl group, a sec-butyl group, a t-butyl group, a neopentyl group, and an n-hexyl group, a cyclohexyl group, and a phenyl group.

Specific examples of the oxygen-containing group include a hydroxy group, an alkoxy group, an aryloxy group, an ester group, an acyl group, a carboxy group, a carbonyl group, and an epoxy group.

Specific examples of the nitrogen-containing group include an amino group, an imino group, an amide group, an imido group, a hydrazino group, a hydrazono group, a nitro group, a nitroso group, a cyano group, an isocyano group, a cyanic acid ester group, an amidino group, and a diazo group.

Examples of the halogen atom include a fluorine atom, a chlorine atom, a bromine atom, and an iodine atom.

R¹, R², and R³ are preferably an alkyl chain that does not have oxygen-containing groups, nitrogen-containing groups, or halogen atom-containing substituents, and more preferably an alkyl chain having no substituent, from the viewpoint of stabilization.

The hetero atom that may be contained in R⁴ is selected from O, N, S, Si, and P. When R⁴ is an aromatic hydrocarbon chain containing a hetero atom, the number of carbon atoms of the aromatic hydrocarbon is, for example, 4 to 12, more preferably 12. Furthermore, when R⁴ is an aliphatic hydrocarbon chain containing a hetero atom, the number of carbon atoms is 2 to 20, more preferably 4. When R⁴ is an aromatic hydrocarbon chain containing no hetero atoms, the number of carbon atoms of the aromatic hydrocarbon is 6 to 12, more preferably 6. When R⁴ is an aliphatic hydrocarbon chain containing no hetero atoms, the number of carbon atoms is 1 to 10, more preferably 2 to 4.

In the present specification, the term "aromatic hydrocarbon" refers to what is preferably an aromatic hydrocarbon of 6 to 12 carbon atoms, and examples thereof include, but are not limited to, mono- and diphenylenes such as 1,2-, 1,3-, and 1,4-phenylene, biphenyl 2,2-yl, biphenyl 3,3-yl, and biphenyl 4,4-yl. Among these, 1,2-, 1,3-, and 1,4-phenylene and the like are preferred from the viewpoint of availability, and 1,4-phenylene is more preferred. When R⁴ is an aromatic hydrocarbon chain containing a hetero atom, examples thereof include groups containing two aromatic chains that each contain an aromatic ring (the number of carbon atoms of each aromatic ring is 6 to 12) and a hetero atom in between. Specific examples thereof include, but are not limited to, a diaryl ether chain, a diarylamine chain, a diaryl sulfide chain, a diaryl phosphine chain, and a diaryl silane chain, such as oxybis(2,1-phenylene), oxybis(3,1-phenylene), oxybis(4,1-phenylene), azadi(1,4-phenylene), azadi(1,3-phenylene), azadi(1,2-phenylene), thiobis(4,1-phenylene), thiobis(3,1-phenylene), thiobis(2,1-phenylene), dimethylsilylenebis(4,1-phenylene), and phenylphosphanylenebis(4,1-phenylene). In this case, the two aromatic rings (aromatic chains) may be different from each other. Among these, oxybis(2,1-phenylene), oxybis(3,1-phenylene), oxybis(4,1-phenylene), and the like are preferred from the viewpoint of availability, and oxybis(4,1-phenylene) is more preferred.

When R⁴ is an aromatic hydrocarbon chain containing a hetero atom, examples thereof also include an aromatic chain (heteroaromatic chain) containing an aromatic ring, called a heteroaromatic (a furan, a thiophene, a pyrrole, a pyridine, etc.). The number of carbon atoms of these aromatic rings is 4 or 5. Examples of the heteroaromatic chains include furan-2,3-diyl, furan-2,4-diyl, furan-2,5-diyl, pyridine-2,3-yl, pyridine-2,4-yl, pyridine-2,5-yl, pyridine-2,6-yl, pyridine-3,4-yl, pyridine-3,5-yl, pyrrolo-2,3-diyl, pyrrolo-2,4-diyl, pyrrolo-2,5-diyl, pyrrolo-3,4-diyl, thieno-2,3-diyl, thieno-2,4-diyl, thieno-2,5-diyl, and thieno-3,4-diyl. Among these, from the viewpoint of availability and stability, furan-2,3-diyl, furan-2,4-diyl, furan-2,5-diyl, thieno-2,3-diyl, thieno-2,4-diyl, thieno-2,5-diyl, thieno-3,4-diyl and the like are preferred, and furan-2,5-diyl, thieno-2,5-diyl and the like are more preferred.

In the present specification, the "aliphatic hydrocarbon" is preferably a linear alkyl chain of 1 to 10 carbon atoms, more preferably 2 to 4 carbon atoms, and examples thereof include, but are not limited to, an alkylene group such as methylene, 1,2-ethylene, 1,3-propylene, butane-1,4-diyl, penta-1,5-diyl, and hexa-1,6-diyl. Among these, from the viewpoint of availability, 1,2-ethylene, 1,3-propylene, butane-1,4-diyl, and the like are preferred, and butane-1,4-diyl is more preferred. When R⁴ is an aliphatic hydrocarbon chain containing a hetero atom, examples thereof include those containing two alkyl chains of 1 to 10 carbon atoms (preferably 2 carbon atoms) and a hetero atom in between (in this case, the number of carbon atoms is the total of the two alkyl chains, and is 2 to 20 (preferably 4 carbon atoms)). Specific examples thereof include, but are not limited to, an alkylene group containing heteroatoms or a dialkyl ether chain, a dialkyl amine chain, a dialkyl sulfide chain, a dialkyl phosphine chain, and a dialkyl silane chain, such as 2-oxapropane-1,3-diyl, 3-oxapentane-1,5-diyl, 4-oxaheptane-1,7-diyl, 2-azapropane-1,3-diyl, 3-azapentane-1,5-diyl, 4-azaheptane-1,7-diyl, 2-thiapropane-1,3-diyl, 3-thiapentane-1,5-diyl, 4-thiaheptane-1,7-diyl, 2-silapropane-1,3-diyl, 3-silapentane-1,5-diyl, 4-silaheptane-1,7-diyl, 2-phosphapropane-1,3-diyl, 3-phosphapentane-1,5-diyl, and 4-phosphaheptane-1,7-diyl. The two alkyl chains may be different from each other. Among these, 2-oxapropane-1,3-diyl, 3-oxapentane-1,5-diyl, 4-oxaheptane-1,7-diyl, and the like are preferred from the viewpoint of ease of handling, and 2-oxapropane-1,3-diyl is more preferred.

From the viewpoint of improving the reaction rate when used as a PA polymerization initiator, R⁴ is preferably a group containing two aromatic hydrocarbon chains of 6 carbon atoms and a hetero atom in between or an aromatic hydrocarbon chain having 6 carbon atoms and containing no hetero atoms. In order to improve solubility in a cyclic lactam when used as an initiator, R⁴ is more preferably a group containing two aromatic hydrocarbon chains of 6 carbon atoms and a hetero atom in between.

An embodiment of the multi-block copolymer according to the present invention is a novel compound represented by the following formula (2): 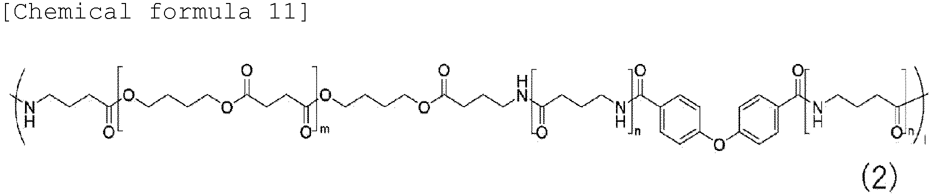 (in the formula (2), m represents an integer of 1 to 60, n represents an integer of 1 to 120, and l represents an integer of 2 to 100).

Such a multi-block copolymer has a number-average molecular weight of, for example, 5,000 to 2,000,000, and preferably 8,000 to 50,000.

The multi-block copolymer of the embodiment is a multi-block copolymer in which multiple bonded bodies each obtained by bonding a polyester block and a polyamide block at 1:1 are continuously lined. For example, when the polyester side is of polybutylene succinate (also referred to as PBS) and the polyamide side is of polyamide 4 (also referred to as PA4), multiple bonded bodies are each obtained by bonding a block containing the polybutylene succinate to a block containing the polyamide 4 at 1:1, as represented by the above-mentioned formula (2), and these multiple bonded bodies are linked in a multi-block copolymer. Therefore, the multi-block copolymer as described herein has l (alphabet "l") as the number of repeating units of 2 or more. An AB-type diblock copolymer or an ABA-type triblock copolymer that has l of 1 is not referred to as a multi-block copolymer.

Polyamide 4 is a rare polyamide among the above-described polyamides in terms of its excellent biodegradability. However, since the melting point (264°C) and the decomposition temperature (270 to 280°C) are close to each other, the moldability is inferior (source: Japanese Patent No. 5988049). On the other hand, a polybutylene succinate exhibits high biodegradability, and has high utility value due to excellent mechanical properties, high moldability, and stability. Therefore, the multi-block copolymer of a polyamide 4 and a polybutylene succinate can achieve a material having excellent biodegradability and moldability.

In the present specification, an aliphatic polyester resin is a polymer in which aliphatic alkyl chains (R¹ and R²) are linked by ester bonds, and examples thereof include a polybutylene succinate, a polybutylene adipate, and a polybutylene succinate adipate. In the present specification, the polyamide resin is a polymer that has an amide bond obtained through ring-opening polymerization of a cyclic lactam.

Specific examples of cyclic lactams include β-propiolactam, γ-butyrolactam, ε-caprolactam, ω-enantolactam, and ω-laurolactam. Examples of the polyamide resin include a poly(butyrolactam) (nylon 4), a polycaprolactam (nylon 6), a polyenantolactam (nylon 7), a polycapryllactam (nylon 8), a polynonanolactam (nylon 9), a polyundecanolactam (nylon 11), and a polylauryllactam (nylon 12).

One aspect of the present invention provides a method for producing a multi-block copolymer represented by the formula (1), including a step of bonding a polyester-containing compound represented by the following formula (3): 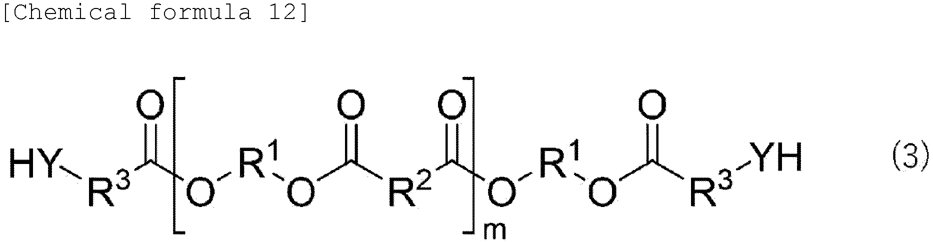 (in the formula (3), R¹ and R² each represent an alkyl chain of 1 to 20 carbon atoms having hydrogen atoms or substituents, and R³ represents an alkyl chain of 1 to 10 carbon atoms having hydrogen atoms or substituents; R¹, R², and R³ may be the same as or different from one another; Y is NH, O (oxygen), or S (sulfur); and m represents an integer of 1 to 60) to
a polyamide-containing compound represented by the following formula (4): 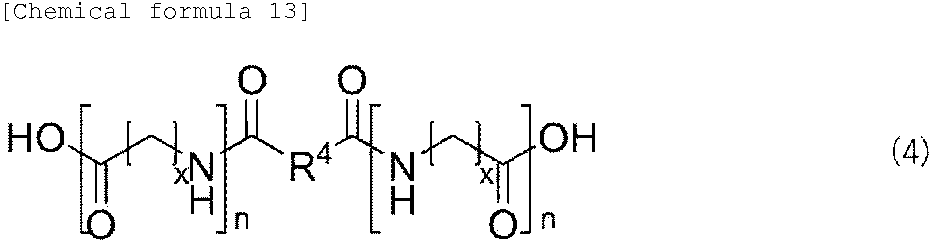
by forming a bond between a YH group in the compound represented by the above-mentioned formula (3) and a carboxy group in the compound represented by the above-mentioned formula (4).
(in the formula (4), R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 12 carbon atoms containing no hetero atoms, and an aliphatic hydrocarbon chain of 1 to 10 carbon atoms containing no hetero atoms; and n represents an integer of 1 to 120, and x represents an integer of 1 to 11).

In an embodiment of the compound represented by the formula (3), when the YH group is an amino group, the compound is represented by the following formula (5). The amino group may be in the form of a salt such as a hydrochloride, sulfate or nitrate, as shown in the following formula (7). Y is preferably NH (the YH group is an amino group) from the viewpoint of the reaction rate with a carboxylic acid. 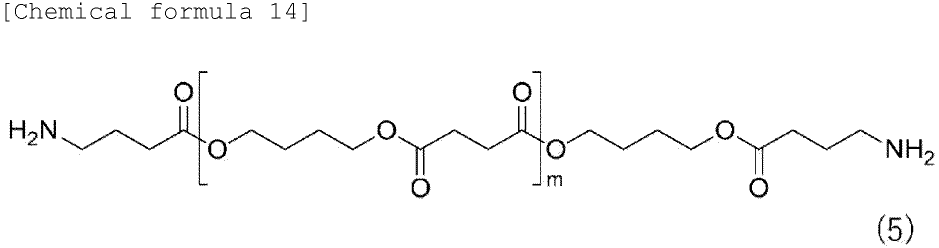 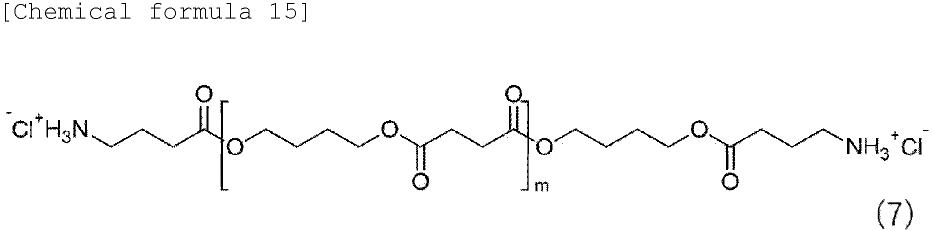 (In the formulas (5) and (7), m is an integer of 1 to 60.)

The ratio of the compound represented by the formula (3) to the compound represented by the formula (4), these compounds being used in the reaction, may be a molar ratio of 1:1.

The reaction is preferably performed under the conditions of being in the presence of a condensing agent. Specific examples of the condensing agent that can be used in the reaction include, but are not limited to, a carbodiimide-based condensing agent. Examples thereof include N,N-diisopropylcarbodiimide (DIC), 1-[3-(dimethylamino)propyl]-3-ethylcarbodiimide (EDCI), and 1-[3-(dimethylamino)propyl]-3-ethylcarbodiimide hydrochloride (EDCI·HCl). Other examples include carbonyldiimidazole (CDI), 4-(4,6-dimethoxy-1,3,5-triazin-2-yl)-4-methylmorpholinium chloride (DMTMM), (1-cyano-2-ethoxy-2-oxoethylidenaminooxy)dimethylamino-morpholino-carbenium hexafluorophosphate (COMU), and 1-[bis(dimethylamino)methylene]-1H-1,2,3-triazolo[4,5-b]pyridinium 3-oxido hexafluorophosphate (HATU).

The usage amount of the condensing agent may be 1 to 5 molar equivalents relative to the carboxy group in the compound represented by the formula (3).

As the solvent used for the reaction, it is preferable to use a solvent that dissolves both the polyester represented by the formula (3) and the polyamide represented by the formula (4), and specific examples thereof include, but are not limited to, N,N-dimethylformamide, N-methylpyrrolidone, and N,N-dimethylacetamide.

Salts are preferably added to the solvent in order to promote dissolution of the polyamide, and specific examples thereof include lithium chloride, lithium bromide, lithium iodide, and calcium chloride.

The salt can be added at a concentration of 10 to 200 g/L relative to the solvent.

When the YH group is an amino group, a base can be added to the reaction as needed to activate the amine-terminus. Specific examples thereof include triethylamine, N,N-diisopropylethylamine, pyridine, 2,6-lutidine, 2,4,6-collidine, and N,N-dimethyl-4-aminopyridine (DMAP).

The base may be added in an amount of 1 to 10 molar equivalents relative to the compound represented by the formula (3) (for example, a compound in which the YH group is an amino group).

The reaction can be performed under conditions of about 0 to 150°C, and more preferably about 80 to 120°C. The reaction can be performed under conditions in which a reaction time is 0.5 to 6.0 hours.

Then, the solution after the reaction is added dropwise to acetone to stop the reaction, thereby obtaining a precipitate. The obtained precipitate is filtered and collected, washed with pure water, and then vacuum-dried to obtain a desired multi-block copolymer.

When the YH group is an amino group, the compound represented by the formula (3) is obtained by introducing an R³CO-NH₂ group into the OH terminal of the compound represented by the following formula (8) (an OH-terminated polyester). For example, the compound represented by the formula (3) can be obtained by, after the introduction of an R³CO-NH-Boc group, removing a Boc group. When Y is O or S, the compound can be obtained by, for example, after the introduction of an R³CO-Y-tert-Bu group, removing a tert-Bu group. R³ represents an alkyl chain of 1 to 10 carbon atoms having hydrogen atoms or substituents. 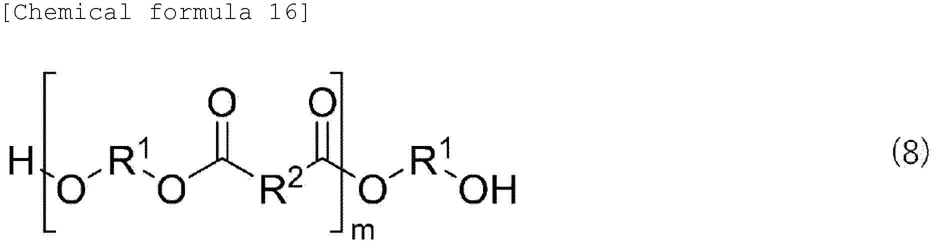 (In the formula (8), R¹ and R² each represent an alkyl chain of 1 to 20 carbon atoms having hydrogen atoms or substituents, and may be the same as or different from each other. Furthermore, m represents an integer of 1 to 60).

The R³CO-NH-Boc group can be introduced, for example, by reacting the compound of the formula (8) with an N-Boc protected amino acid. Specific examples of the N-Boc protected amino acid include 4-(tert-butoxycarbonylamino)butyric acid, 5-(tert-butoxycarbonylamino)pentanoic acid, and 6-(tert-butoxycarbonylamino) hexanoic acid. In the reaction, the compound represented by the formula (8) and the N-Boc protected amino acid are preferably reacted at a molar ratio of 1:1 to 1:5.

For the reaction, for example, a solvent such as dichloromethane, chloroform, or a mixed solvent thereof can be used. As the condensing agent, a carbodiimide-based condensing agent can be used. Examples thereof include N,N-diisopropylcarbodiimide (DIC), 1-[3-(dimethylamino)propyl]-3-ethylcarbodiimide (EDCI), and 1-[3-(dimethylamino)propyl]-3-ethylcarbodiimide hydrochloride (EDCI·HCl). The usage amount of the condensing agent may be 1 to 5 molar equivalents relative to the N-Boc protected amino acid.

As a condensation aid, N,N-dimethyl-4-aminopyridine (DMAP), 1-hydroxybenzotriazole, or 1-hydroxy-7-azabenzotriazole may be added to the reaction as needed. The amount of the condensation aid added may be about 0 to 2 molar equivalents relative to the N-Boc protected amino acid.

The reaction can be performed under conditions of about -20°C to 60°C, more preferably, about 0 to 40°C. The reaction can be performed under conditions in which a reaction time is 1 to 4 hours. In the reaction solution obtained after the reaction, pure water is added to stop the reaction. Then, the reaction solution is concentrated, washed, and dried to obtain a compound represented by the formula (9) (a both-ends NH-Boc-terminated polyester), for example, as a white solid. When Y is O or S, the compound can be similarly synthesized using a condensing agent such as DIC, CDI, or the like, and a base such as triethylamine, N,N-diisopropylethylamine, pyridine, 2,6-lutidine, 2,4,6-collidine, or N,N-dimethyl-4-aminopyridine (DMAP). 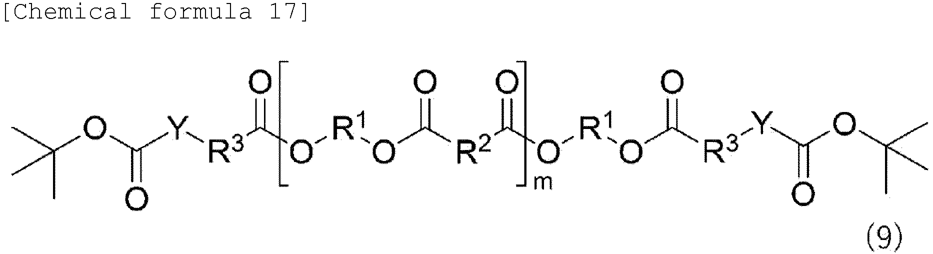 (in the formula (9), R¹ and R² each represent an alkyl chain of 1 to 20 carbon atoms having hydrogen atoms or substituents, and R³ represents an alkyl chain of 1 to 10 carbon atoms having hydrogen atoms or substituents. R¹, R² and R³ may be the same as or different from one another. Y is NH. Furthermore, m represents an integer of 1 to 60).

The removal of the Boc group of the both-ends NH-Boc-terminated polyester can be performed according to a known method and can be performed by adding a strong acid such as ethyl acetate containing hydrogen chloride or 1,4-dioxane containing hydrogen chloride to obtain a salt-type compound that has a neutralized terminal amino group and is represented by the formula (10). Alternatively, the compound represented by the formula (11) can be obtained by subjecting the compound to a base treatment with sodium bicarbonate or the like. 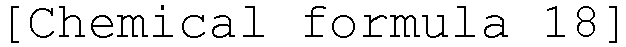 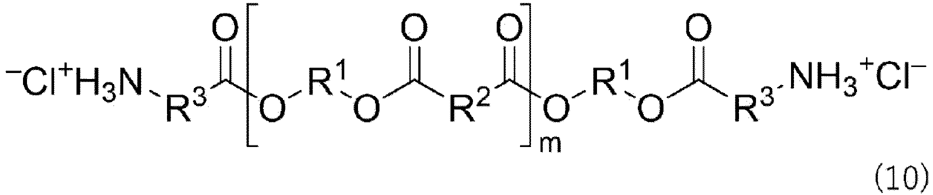 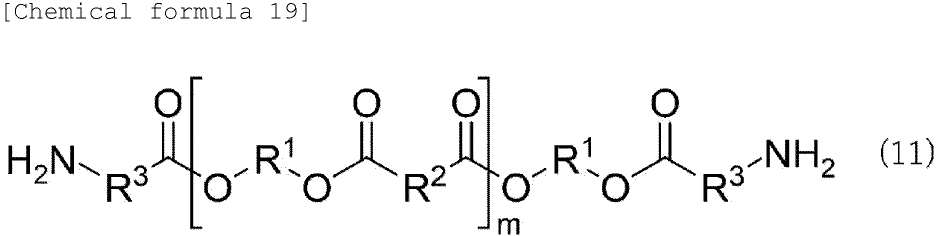 (in the formulas (10) and (11), R¹ and R² each represent an alkyl chain of 1 to 20 carbon atoms having hydrogen atoms or substituents, and R³ represents an alkyl chain of 1 to 10 carbon atoms having hydrogen atoms or substituents. R¹, R² and R³ may be the same as or different from one another. Furthermore, m represents an integer of 1 to 60).

The compound represented by the above-mentioned formula (8) (OH-terminated polyester) can be produced by reacting a divalent alcohol (OH-R¹-OH) (R¹ is an alkyl chain of 1 to 20 carbon atoms having hydrogen atoms or substituents) with a dicarboxylic acid (COOH-R²-COOH) (R² is an alkyl chain of 1 to 20 carbon atoms having hydrogen atoms or substituents).

As a divalent alcohol for use in the reaction step, for example, ethylene glycol, 1,3-propanediol, 1,4-butanediol, and the like can be mentioned. As the dicarboxylic acid used, malonic acid, succinic acid, glutaric acid, sebacic acid, adipic acid, and the like can be mentioned.

The blend ratio of the divalent alcohol and the dicarboxylic acid for use in the reaction step can be a molar ratio of 1:1 to 2:1. Since the divalent alcohol added in an excess amount serves as an end-capping agent, the degree of polymerization of the polyester block can be adjusted by appropriately selecting the blend ratio.

As the solvent in the reaction step, a halogen-based organic solvent such as dichloromethane, chloroform, or dichloroethane, or a mixed solvent thereof can be used.

As the condensing agent in the reaction, a carbodiimide-based condensing agent can be used, and examples thereof include N,N-diisopropylcarbodiimide (DIC), 1-[3-(dimethylamino)propyl]-3-ethylcarbodiimide (EDCI), and 1-[3-(dimethylamino)propyl]-3-ethylcarbodiimide hydrochloride (EDCI·HCl).

The usage amount of the condensing agent may be about 1 to 5 molar equivalents relative to the hydroxycarboxylic acid.

As a condensation aid, N,N-dimethylaminopyridine (DMAP), 1-hydroxybenzotriazole, or 1-hydroxy-7-azabenzotriazole may be added to the reaction as needed. The amount of the condensation aid added may be about 0 to 2 molar equivalents relative to the hydroxycarboxylic acid.

The reaction can be performed under conditions of about -20°C to 60°C, and more preferably about 0 to 40°C. The reaction can be performed under conditions in which a reaction time is 1 to 4 hours. In the reaction solution obtained after the reaction, pure water is added to stop the reaction. Then, the reaction solution is concentrated and dried to obtain a compound represented by the formula (8) (OH-terminated polyester), for example, as a white solid.

The compound represented by the formula (4) is obtained through ring-opening polymerization of a compound represented by the formula (12) (PA polymerization reaction initiator) and a cyclic lactam in the presence of a base, and subsequently hydrolysis of the terminal lactam: 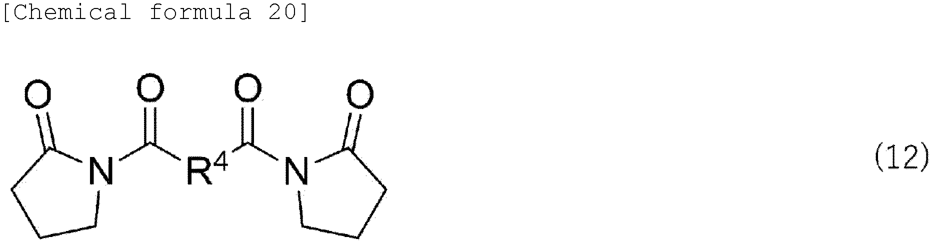 (In the formula (12), R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 12 carbon atoms containing no hetero atoms, and an aliphatic hydrocarbon chain of 1 to 10 carbon atoms containing no hetero atoms).

As the cyclic lactam, a cyclic lactam of 2 to 12 carbon atoms can be used. As described above, specific examples thereof include β-propiolactam, γ-butyrolactam, ε-caprolactam, ω-enantolactam, and ω-laurolactam.

The blend ratio of the PA polymerization reaction initiator represented by the formula (12) for use in the reaction to the cyclic lactam may be a molar ratio of 1:1 to 1:240. Since the compound of the formula (12) acts as an initiator for ring-opening polymerization, the degree of polymerization of the polyamide block can be adjusted by appropriately selecting the blend ratio.

As the base, a metal alkoxide such as sodium tert-butoxide or potassium tert-butoxide, or a metal salt of a cyclic lactam used for polymerizing 2-pyrrolidone sodium salt or the like can be used. The amount of the base used may be 0.01 to 1 molar equivalents relative to the cyclic lactam.

The reaction is preferably performed in an inert gas atmosphere such as that with an argon gas.

The reaction can be performed under conditions of about 0 to 60°C, and more preferably about 0 to 40°C. The reaction can be performed under conditions in which a reaction time is 1 to 24 hours. The solid matter obtained after the reaction is dissolved in concentrated hydrochloric acid or the like to hydrolyze the terminal lactam, and then subjected to concentration and drying steps, whereby a compound represented by the formula (4) (both-ends carboxy group-terminated PA) can be obtained, for example, as a white solid.

The PA polymerization reaction initiator of the formula (12) can be synthesized by a condensation reaction, according to a known method, using the corresponding lactam or a derivative thereof and a dicarboxylic acid, a dicarboxylic acid chloride, or a derivative thereof.

The following chemical reaction formulas (13) and (14) show examples of reaction formulas when the polyamide side is PA4 and the polyester side is PBS with respect to the above-described reaction. Furthermore, the following chemical reaction formula (15) shows an example of a reaction formula in which a multi-block copolymer (compound of the formula (2)) is produced using a polyamide component (compound of the formula (6)) and a polyester component (compound of the formula (7)), which are products in the chemical reaction formula (13) and the chemical reaction formula (14), respectively. By adjusting the blend ratio of the polyamide 4 component and the polyester component, the physical properties can be adjusted according to the usages. For example, when the heat resistance is enhanced, the polyamide component ratio in the macromolecular may be increased by copolymerizing a polyamide having a high average degree of polymerization and a polyester having a low average degree of polymerization.

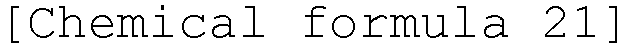

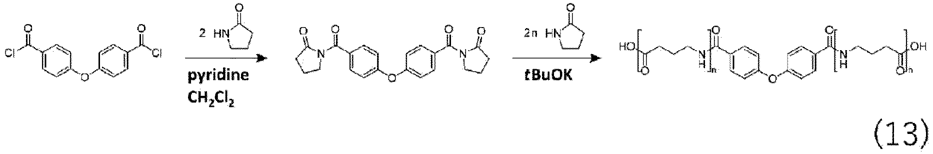

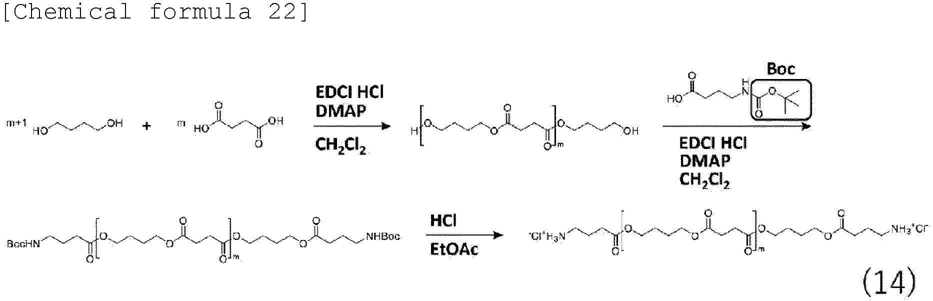

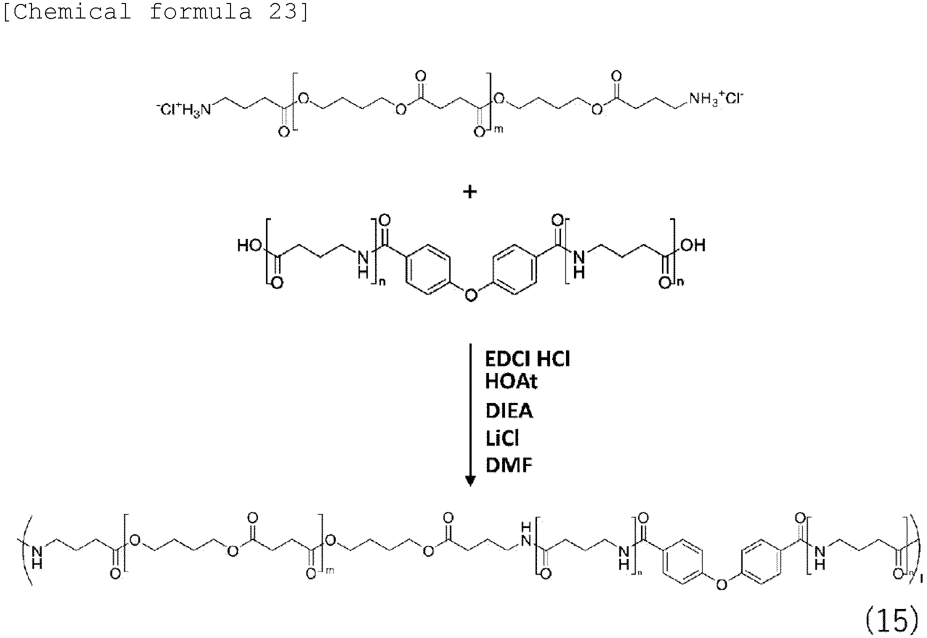

In another embodiment, the compound represented by the formula (6) that is to be reacted with the compound represented by the formula (7) may be replaced with 4,4'-oxybisbenzoic acid represented by the following formula (20).

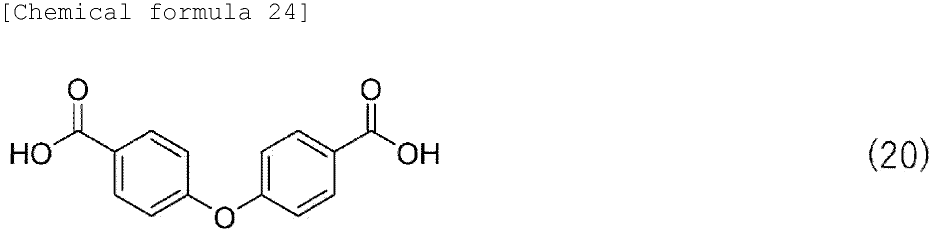

In such a case, in the multi-block copolymer of the formula (2), an ester-amide copolymer with n = 0 (compound of the following formula (19)) can be obtained. 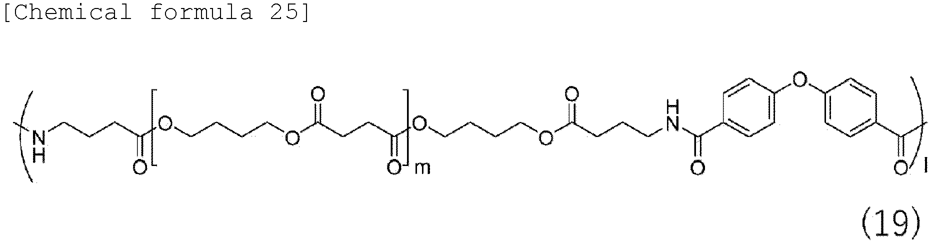 (in the formula (19), m represents an integer of 1 to 60, and l represents an integer of 2 to 100).
The chemical reaction formula (21) in this case is shown below.

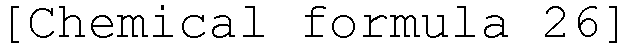

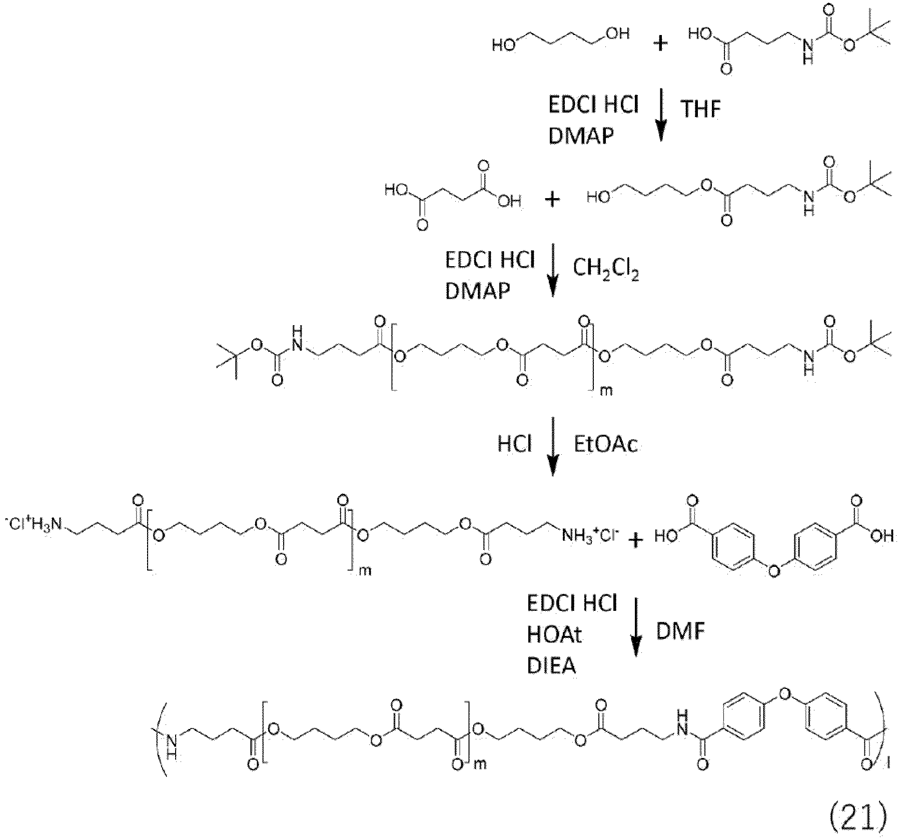

### [Examples]

Hereinafter, embodiments of the present invention will be described in detail with reference to Examples according to the chemical reaction formulas (13) to (15) described above, but the present invention is not limited to these embodiments.

### <Example 1>

A multi-block copolymer was synthesized through a reaction step (step 6) for a reaction between a both-ends amino group-terminated PBS and a both-ends carboxy group-terminated PA4. These reactants are synthesized by synthesis steps (steps 1 to 3) for the both-ends amino group-terminated PBS and synthesis steps (steps 4 to 5) for the both-ends carboxy group-terminated PA4, respectively. Whether the synthesis step for the both-ends amino group-terminated PBS or the synthesis step for the both-ends carboxy group-terminated PA4 is performed first does not matter.

[Synthesis of both-ends amino group-terminated PBS] Step 1 (Synthesis of both-ends hydroxyl group-terminated PBS (compound represented by the following formula (16)))

### (Experiment Examples 1 to 5)

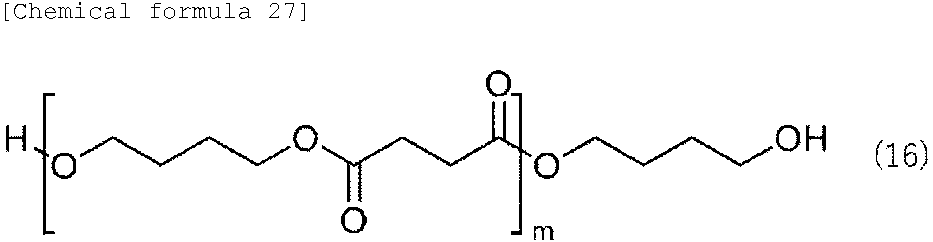

### (Experiment Example 1)

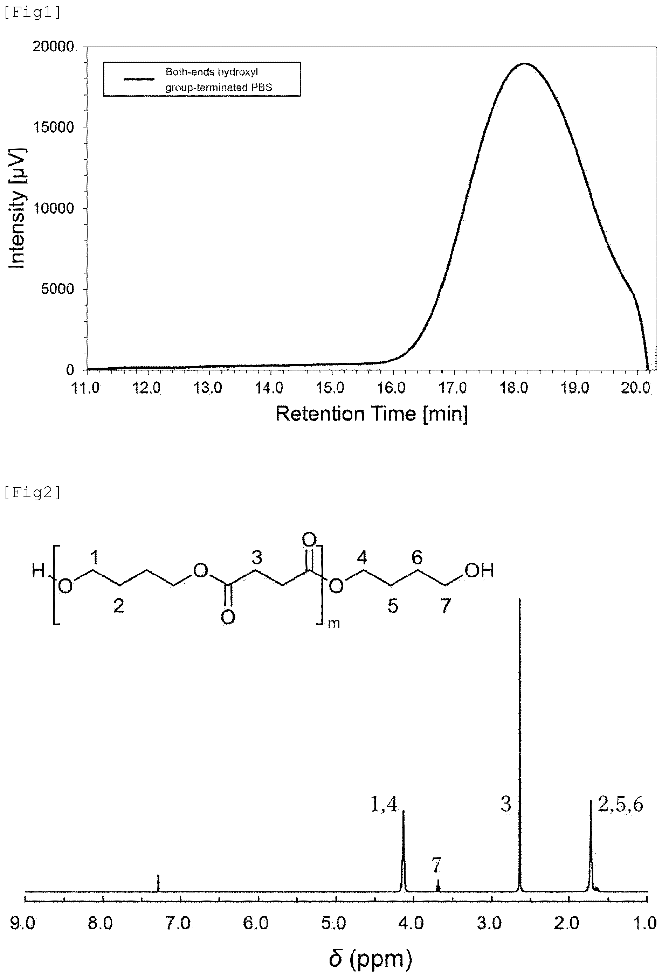

Into a 500 mL recovery flask, 1,4-butanediol (manufactured by FUJIFILM Wako Pure Chemical Corporation, 8.395 g, 93.15 mmol), succinic acid (manufactured by FUJIFILM Wako Pure Chemical Corporation, 10.00 g, 84.68 mmol), DMAP (manufactured by FUJIFILM Wako Pure Chemical Corporation, 4.345 g, 35.57 mmol), and dichloromethane (manufactured by FUJIFILM Wako Pure Chemical Corporation, 267 mL) were placed. The mixture was cooled in an ice bath, and then added with EDCI·HCl (manufactured by Peptide Institute, Inc., 51.138 g, 266.75 mmol). The mixture was stirred for 30 minutes, and then further stirred at room temperature for 3 hours. Thereafter, 50 mL of pure water was added to terminate the reaction. The resultant mixed solution was concentrated, and the residue was washed with 200 mL of methanol twice and with 200 mL of pure water once and then vacuum dried at 45°C to obtain a target product (both-ends hydroxyl group-terminated PBS) as a white solid. The yield amount was 11.521 g, and the yield was 75%. The molecular weight distribution measurement was performed by GPC, and Mn and Mw were calculated as 4.5 kDa and 10.2 kDa, respectively (Fig. 1). The average degree of polymerization was calculated to be m = 15 from the peak area ratio between 7 and 3 of the ¹H-NMR spectrum shown in Fig. 2. In the following Experiment Examples, the same reagent and the same apparatus were used for the parts that correspond with Experiment Example 1, unless otherwise stated.

### (Experiment Example 2)

A both-ends hydroxyl group-terminated PBS was obtained by the methods described in Experiment Example 1. The yield amount was 10.674 g and the yield was 70%. From the ¹H-NMR spectrum, the average degree of polymerization was calculated to be m = 14.

### (Experiment Example 3)

A both-ends hydroxyl group-terminated PBS was obtained by the same methods as described in Experiment Example 1, except that 1,4-butanediol (8.013 g, 88.92 mmol), succinic acid (10.00 g, 84.68 mmol), DMAP (4.242 g, 34.72 mmol), dichloromethane (267 mL), and EDCI·HCl (49.920 g, 260.40 mmol) were used. The yield amount was 11.319 g and the yield was 76%. From the ¹H-NMR spectrum, the average degree of polymerization was calculated to be m = 17.

### (Experiment Example 4)

A both-ends hydroxyl group-terminated PBS was obtained by the same methods as described in Experiment Example 1, except that 1,4-butanediol (7.886 g, 87.50 mmol), succinic acid (10.00 g, 84.68 mmol), DMAP (4.207 g, 34.44 mmol), dichloromethane (267 mL), and EDCI·HCl (49.514 g, 258.28 mmol) were used. The yield amount was 11.735 g and the yield was 79%. From the ¹H-NMR spectrum, the average degree of polymerization was calculated to be m = 31.

### (Experiment Example 5)

A both-ends hydroxyl group-terminated PBS was obtained by the same methods as described in Experiment Example 1, except that 1,4-butanediol (7.759 g, 86.09 mmol), succinic acid (10.00 g, 84.68 mmol), DMAP (4.173 g, 34.15 mmol), dichloromethane (267 mL), and EDCI·HCl (49.109 g, 256.16 mmol) were used. The yield amount was 12.016 g and the yield was 81%. From the ¹H-NMR spectrum, the average degree of polymerization was calculated to be m = 50.

Step 2 (Synthesis of both-ends NH-Boc-terminated PBS (compound represented by the following formula (17)))

### (Experiment Examples 6 to 10)

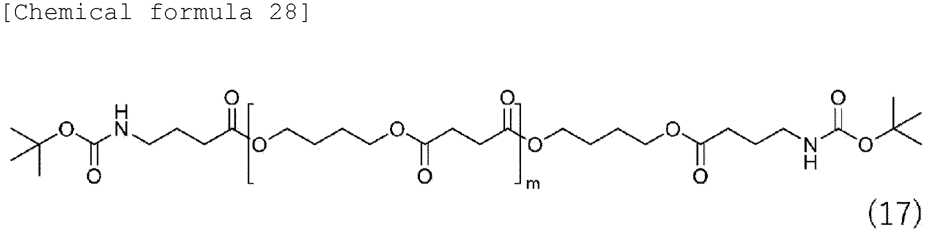

### (Experiment Example 6)

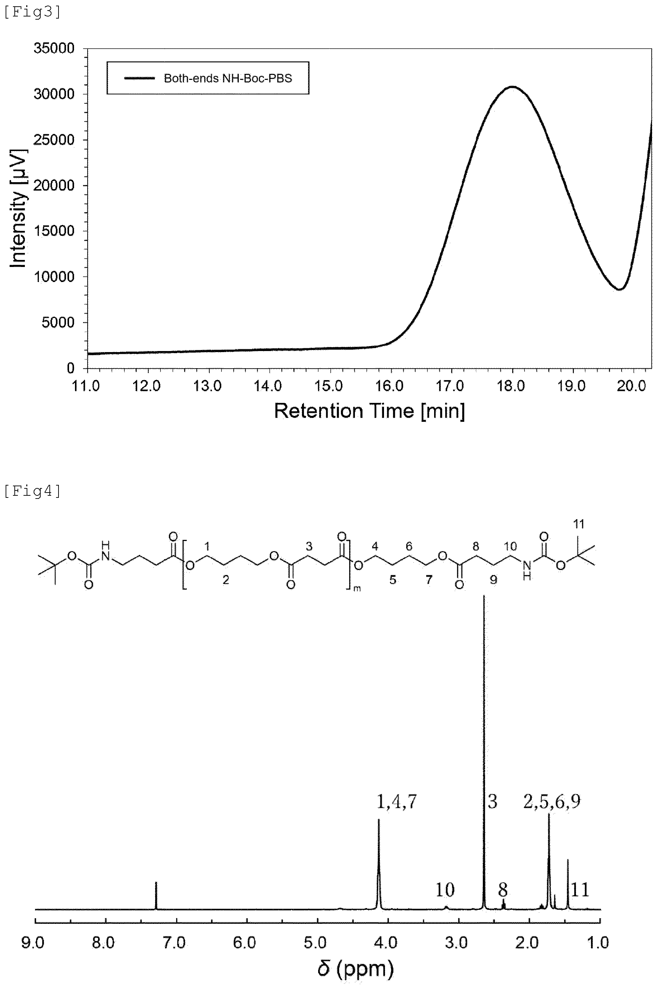

Into a 200 mL recovery flask, the both-ends hydroxyl group-terminated PBS (11.000 g, 4.16 mmol) obtained in Experiment Example 1, DMAP (0.325 g, 2.66 mmol), dichloromethane (110 mL), and 4-(tert-butoxycarbonylamino)butyric acid (manufactured by Tokyo Chemical Industry Co., Ltd., 1.859 g, 9.15 mmol) were placed and dissolved. EDCI·HCl (3.827 g, 19.96 mmol) was added to the mixture under ice cooling, and the mixture was stirred for 30 minutes. The mixture was further stirred at room temperature for 3 hours, and then 50 mL of pure water was added to terminate the reaction. The resultant mixed solution was concentrated, and the residue was washed with 200 mL of methanol twice and with 200 mL of pure water once, and then vacuum dried at 45°C to obtain a target product (both-ends NH-Boc-terminated PBS) as a white solid. The yield amount was 11.878 g, and the yield was 95%. The molecular weight distribution measurement was performed by GPC, and Mn and Mw were calculated as 5.5 kDa and 12.0 kDa, respectively (Fig. 3). The average degree of polymerization was calculated to be m = 15 from the peak area ratio between 10 and 3 of the ¹H-NMR spectrum shown in Fig. 4.

### (Experiment Example 7)

A both-ends NH-Boc-terminated PBS was obtained by the same methods as in Experiment Example 6, except that the both-ends hydroxyl group-terminated PBS (10.000 g, 3.94 mmol) obtained in Experiment Example 2, DMAP (0.308 g, 2.52 mmol), dichloromethane (100 mL), 4-(tert-butoxycarbonylamino)butyric acid (1.764 g, 8.68 mmol), and EDCI·HCl (3.630 g, 18.93 mmol) were used. The yield amount was 10.860 g and the yield was 87%. From the ¹H-NMR spectrum, the average degree of polymerization was calculated to be m = 16.

### (Experiment Example 8)

A both-ends NH-Boc-terminated PBS was obtained by the same methods as in Experiment Example 6, except that the both-ends hydroxyl group-terminated PBS (11.295 g, 3.80 mmol) obtained in Experiment Example 3, DMAP (0.297 g, 2.43 mmol), dichloromethane (120 mL), 4-(tert-butoxycarbonylamino)butyric acid (1.699 g, 8.36 mmol), and EDCI·HCl (3.497 g, 18.24 mmol) were used. The yield amount was 12.106 g and the yield was 96%. From the ¹H-NMR spectrum, the average degree of polymerization was calculated to be m = 18.

### (Experiment Example 9)

A both-ends NH-Boc-terminated PBS was obtained by the same methods as in Experiment Example 6, except that the both-ends hydroxyl group-terminated PBS (11.735 g, 2.16 mmol) obtained in Experiment Example 4, DMAP (0.170 g, 1.39 mmol), dichloromethane (117 mL), 4-(tert-butoxycarbonylamino)butyric acid (0.966 g, 4.75 mmol), and EDCI·HCl (2.000 g, 10.43 mmol) were used. The yield amount was 11.881 g and the yield was 87%. From the ¹H-NMR spectrum, the average degree of polymerization was calculated to be m = 35.

### (Experiment Example 10)

A both-ends NH-Boc-terminated PBS was obtained by the same methods as in Experiment Example 6, except that the both-ends hydroxyl group-terminated PBS (12.016 g, 1.39 mmol) obtained in Experiment Example 5, DMAP (0.109 g, 0.89 mmol), dichloromethane (120 mL), 4-(tert-butoxycarbonylamino)butyric acid (0.623 g, 3.06 mmol), and EDCI·HCl (1.282 g, 6.686 mmol) were used. The yield amount was 11.986 g and the yield was 95%. From the ¹H-NMR spectrum, the average degree of polymerization was calculated to be m = 51.

Step 3 (Synthesis of both-ends amino group-terminated PBS (compound represented by the following formula (7))) (Experiment Examples 11 to 15)

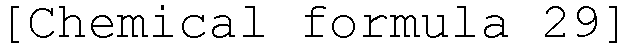

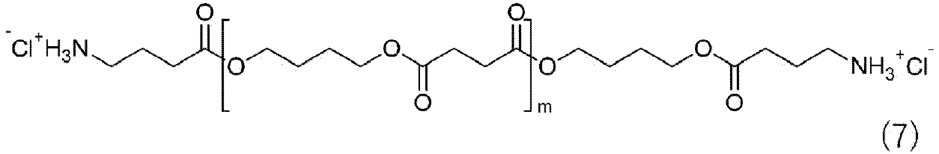

### (Experiment Example 11)

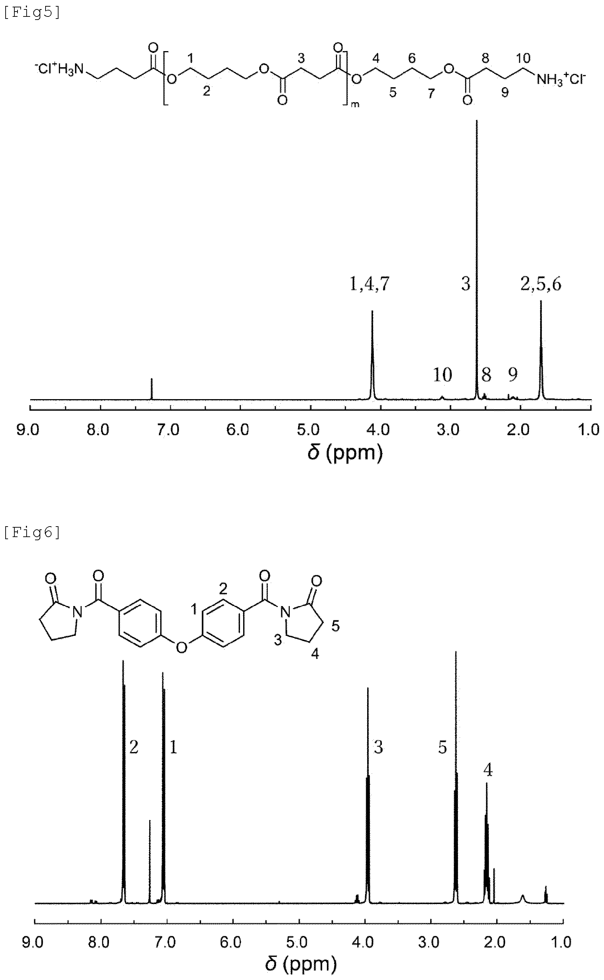

Into a 200 mL recovery flask, the both-ends NH-Boc-terminated PBS (11.500 g, 3.81 mmol) obtained in Experiment Example 6 and a 4M hydrogen chloride/ethyl acetate solution (manufactured by FUJIFILM Wako Pure Chemical Corporation, 58 mL) were placed. The suspension was stirred at room temperature for 3 hours. The reaction solution was dropped to 200 mL of acetone, washed to collect insoluble matters, and vacuum dried at 45°C to obtain a target product (both-ends amino group-terminated PBS) as a white solid. The yield amount was 10.030 g, and the yield was 91%. Since strong tailing, which was considered resulting from adsorption to the column, was observed in the GPC chart, the molecular weight of the both-ends amino group-terminated PBS could not be calculated. On the other hand, the average degree of polymerization was calculated to be m = 15 from the peak area ratio between 10 and 3 of the ¹H-NMR spectrum shown in Fig. 5.

### (Experiment Example 12)

A both-ends amino group-terminated PBS was obtained by the same methods as in Experiment Example 11, except that the both-ends NH-Boc-terminated PBS (10.800 g, 3.43 mmol) obtained in Experiment Example 7 and a 4M hydrogen chloride/ethyl acetate solution (52 mL) were used. The yield amount was 9.997 g and the yield was 94%. From the ¹H-NMR spectrum, the average degree of polymerization was calculated to be m = 17.

### (Experiment Example 13)

A both-ends amino group-terminated PBS was obtained by the same methods as in Experiment Example 11, except that the both-ends NH-Boc-terminated PBS (12.106 g, 3.66 mmol) obtained in Experiment Example 8 and a 4M hydrogen chloride/ethyl acetate solution (60 mL) were used. The yield amount was 11.199 g and the yield was 81%. From the ¹H-NMR spectrum, the average degree of polymerization was calculated to be m = 21.

### (Experiment Example 14)

A both-ends amino group-terminated PBS was obtained by the same methods as in Experiment Example 11, except that the both-ends NH-Boc-terminated PBS (11.882 g, 1.88 mmol) obtained in Experiment Example 9 and a 4M hydrogen chloride/ethyl acetate solution (60 mL) were used. The yield amount was 11.339 g and the yield was 97%. From the ¹H-NMR spectrum, the average degree of polymerization was calculated to be m = 32.

### (Experiment Example 15)

A both-ends amino group-terminated PBS was obtained by the same methods as in Experiment Example 11, except that the both-ends NH-Boc-terminated PBS (11.986 g, 1.33 mmol) obtained in Experiment Example 10 and a 4M hydrogen chloride/ethyl acetate solution (60 mL) were used. The yield amount was 11.558 g and the yield was 98%. From the ¹H-NMR spectrum, the average degree of polymerization was calculated to be m = 53.

[Synthesis of both-ends carboxy group-terminated PA4]

### Step 4 (Synthesis of PA4 polymerization reaction initiator (compound represented by the following formula (18))) (only Experiment Example 16)

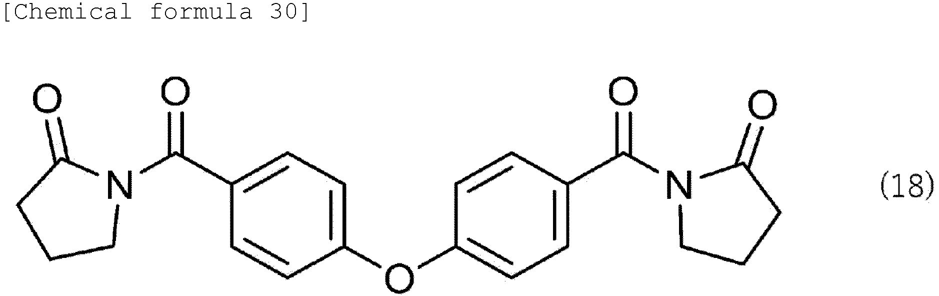

### (Experiment Example 16)

Into a 25 mL recovery flask, pyridine (manufactured by FUJIFILM Wako Pure Chemical Corporation, 1.583 g, 20.00 mmol), 2-pyrrolidone (manufactured by FUJIFILM Wako Pure Chemical Corporation, 2.128 g, 25.00 mmol), and dichloromethane (10 mL) were placed. The mixture was cooled to 0°C. To the reaction mixture, 4,4'-oxybisbenzoyl chloride (manufactured by Tokyo Chemical Industry Co., Ltd., 2.951 g, 10.00 mmol) was added once for all, and the mixture was stirred at room temperature for 3 hours. To the reaction solution, 30 mL of dichloromethane was added to be diluted. The solution was liquid-separated and washed with 30 mL of pure water, 30 mL of a 1M KHSO₄ aqueous solution, and 30 mL of saturated NaHCO₃ in this order. The obtained organic layer was collected, added with MgSO₄ for dehydration, concentrated, and purified by column chromatography (developing solution: gradient of ethyl acetate / dichloromethane = 1/9 to 4/1) to obtain a target product (PA4 polymerization reaction initiator) as a white solid. The yield amount was 1.700 g, and the yield was 44%. Fig. 6 shows the measurement result of a ¹H-NMR spectrum. Since each peak could be assigned to an intended structure, it was confirmed that a target product was obtained.

### Step 5 (Synthesis of both-ends carboxy group-terminated PA4 (compound represented by the following formula (6))) (Experiment Examples 17 to 22)

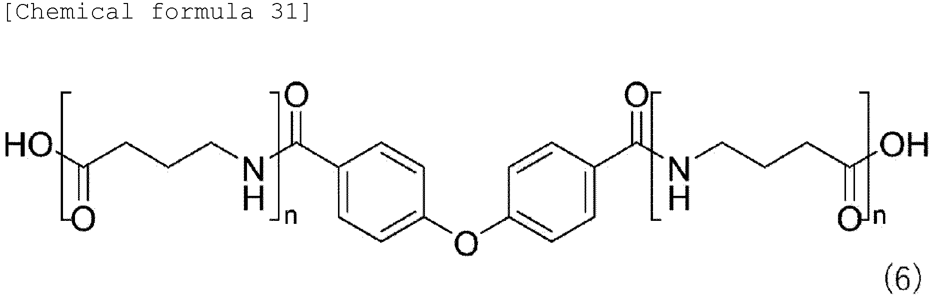

### (Experiment Example 17)

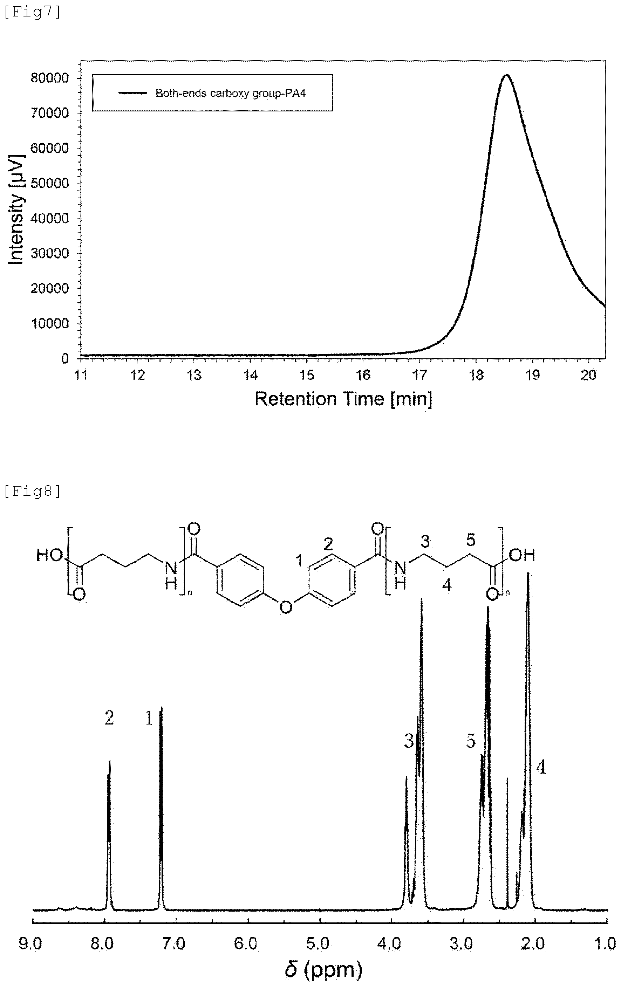

Into a 10 mL recovery flask, the PA4 polymerization reaction initiator (0.508 g, 1.30 mmol) obtained in Experiment Example 16 and 2-pyrrolidone (2.213 g, 26.00 mmol) were placed. The mixture was heated to 90°C and dissolved, and then left to cool to 30°C. To the resultant solution, t-BuOK (manufactured by Tokyo Chemical Industry Co., Ltd., 0.058 g, 0.52 mmol) was added, and the mixture was reacted for 18 hours. During the reaction, the solution gradually solidified, and the stirrer stopped. Into the flask, 7.5 mL of concentrated hydrochloric acid was added and stirred for 20 hours to dissolve the solid matter. The resultant solution was dropped to a mixed solvent of 20 mL of water and 180 mL of acetone to obtain a precipitate. The obtained precipitate was filtered and collected, washed with 100 mL of acetone and 100 mL of pure water in this order, and vacuum dried at 45°C to obtain a target product (both-ends carboxy group-terminated PA4) as a white solid. The yield amount was 0.97 g, and the yield was 37%. The molecular weight distribution measurement was performed by GPC, and Mn and Mw were calculated as 2.0 kDa and 4.7 kDa, respectively (Fig. 7). The average degree of polymerization was calculated as n = 6 from the peak area ratio between 1 and 3 of the ¹H-NMR spectrum shown in Fig. 8.

### (Experiment Example 18)

A both-ends carboxy group-terminated PA4 was obtained by the same methods as in Experiment Example 17, except that the PA4 polymerization reaction initiator (1.562 g, 4.00 mmol) obtained in Experiment Example 16, 2-pyrrolidone (3.404 g, 40.00 mmol), and t-BuOK (0.090 g, 0.80 mmol) were used. The yield amount was 2.719 g and the yield was 61%. From the peak area ratio of the ¹H-NMR spectrum, the average degree of polymerization was calculated to be n = 5.

### (Experiment Example 19)

A both-ends carboxy group-terminated PA4 was obtained by the same methods as in Experiment Example 17, except that the PA4 polymerization reaction initiator (1.100 g, 2.82 mmol) obtained in Experiment Example 16, 2-pyrrolidone (4.796 g, 56.35 mmol), and t-BuOK (0.126 g, 1.13 mmol) were used. The yield amount was 4.10 g and the yield was 68%. From the peak area ratio of the ¹H-NMR spectrum, the average degree of polymerization was calculated to be n = 9.

### (Experiment Example 20)

A both-ends carboxy group-terminated PA4 was obtained by the same methods as in Experiment Example 17, except that the PA4 polymerization reaction initiator (1.073 g, 2.75 mmol) obtained in Experiment Example 16, 2-pyrrolidone (9.362 g, 110.00 mmol), and t-BuOK (0.123 g, 1.10 mmol) were used. The yield amount was 1.47 g and the yield was 26%. From the peak area ratio of the ¹H-NMR spectrum, the average degree of polymerization was calculated to be n = 11.

### (Experiment Example 21)

A both-ends carboxy group-terminated PA4 was obtained by the same methods as in Experiment Example 17, except that the PA4 polymerization reaction initiator (1.757 g, 4.50 mmol) obtained in Experiment Example 16, 2-pyrrolidone (7.660 g, 90.00 mmol), and t-BuOK (0.505 g, 4.50 mmol) were used. The yield amount was 1.78 g and the yield was 15%. From the peak area ratio of the ¹H-NMR spectrum, the average degree of polymerization was calculated to be n = 14.

### (Experiment Example 22)

A both-ends carboxy group-terminated PA4 was obtained by the same methods as in Experiment Example 17, except that the PA4 polymerization reaction initiator (1.400 g, 3.59 mmol) obtained in Experiment Example 16, 2-pyrrolidone (12.207 g, 143.43 mmol), and t-BuOK (0.322 g, 2.87 mmol) were used. The yield amount was 9.40 g and the yield was 68%. From the peak area ratio of the ¹H-NMR spectrum, the average degree of polymerization was calculated to be n = 20.

### Step 6 (Synthesis of PBS-PA4 multi-block copolymer (compound represented by the following formula (2)))

### (Experiment Examples 23 to 33)

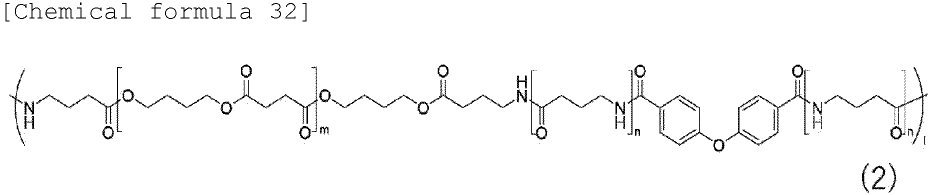

### (Experiment Example 23)

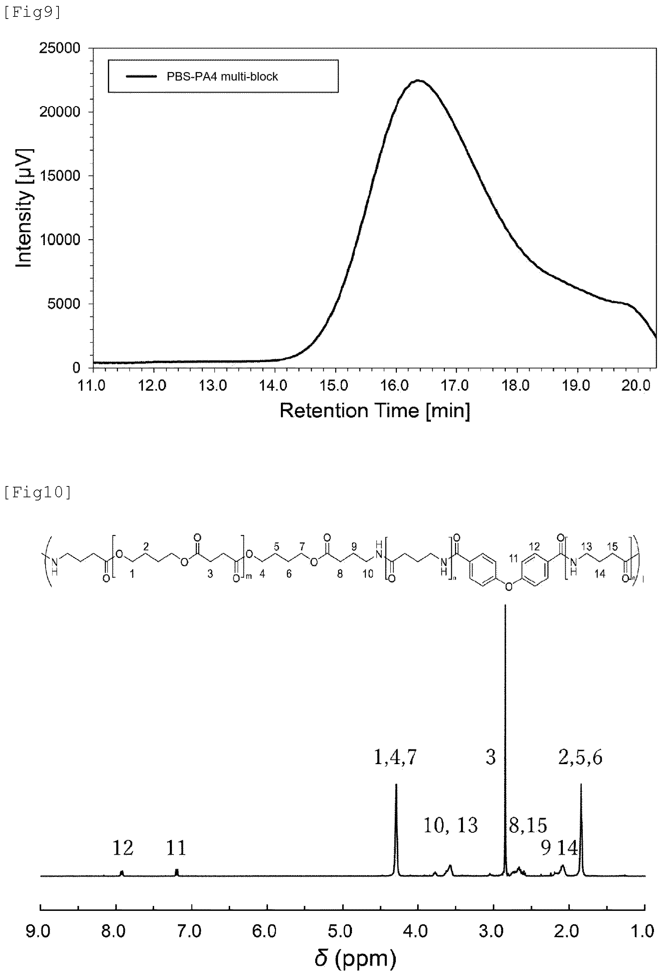

Into a 10 mL recovery flask, the both-ends amino group-terminated PBS (0.200 g, 0.069 mmol) obtained in Experiment Example 11, N,N-diisopropylethylamine (manufactured by FUJIFILM Wako Pure Chemical Corporation, 0.027 g, 0.21 mmol), N,N-dimethylformamide (manufactured by FUJIFILM Wako Pure Chemical Corporation, 1.4 mL), and lithium chloride (manufactured by FUJIFILM Wako Pure Chemical Corporation, 0.140 g, 3.30 mmol) were placed. The mixture was stirred at 100°C for 30 minutes and dissolved. After that, the both-ends carboxy group-terminated PA4 (0.085 g, 0.07 mmol) obtained in Experiment Example 17 was added and further stirred for 30 minutes and dissolved. To the resultant solution, HOAt (manufactured by Tokyo Chemical Industry Co., Ltd., 4.7 mg, 0.03 mmol) and EDCI·HCl (0.029 g, 0.15 mmol) were placed. The mixture was stirred at 100°C for 3 hours. The reaction solution was dropped to 50 mL of acetone to stop the reaction. The precipitate was filtered and collected, washed with 50 mL of pure water, and vacuum dried at 45°C to obtain a white solid (PBS-PA4 multi-block copolymer). The yield amount was 0.23 g, and the yield was 84%. The molecular weight distribution measurement was performed by GPC, and Mn and Mw were calculated as 12.7 kDa and 50.5 kDa, respectively (Fig. 9). Furthermore, from the molecular weight calculated from the ¹H-NMR of each of the both-ends amino group-terminated PBS described in Experiment Example 11 and the both-ends carboxy group-terminated PA4 described in Experiment Example 17, and the Mn of the multi-block copolymer, l (alphabet 'l') was calculated as 3. Furthermore, m was calculated as 16 from the peak area ratio between 11 and 3 of the ¹H-NMR spectrum shown in Fig. 10, and n was calculated as 6 from the peak area ratio between 11, and 10 and 13. This PBS-PA4 multi-block copolymer was defined as sample 1.

### (Experiment Example 24)

A PBS-PA4 multi-block copolymer was obtained by the same methods as in Experiment Example 23, except that the both-ends amino group-terminated PBS (1.500 g, 0.51 mmol) obtained in Experiment Example 11, N,N-diisopropylethylamine (0.133 g, 1.03 mmol), N,N-dimethylformamide (15 mL), lithium chloride (1.500 g, 35.39 mmol), the both-ends carboxy group-terminated PA4 (0.963 g, 0.51 mmol) obtained in Experiment Example 19, HOAt (35 mg, 0.26 mmol), and EDCI·HCl (0.217 g, 1.13 mmol) were used. The yield amount was 2.24 g and the yield was 93%. The molecular weight distribution measurement was performed by GPC, and Mn and Mw were calculated as 15.1 kDa and 36.6 kDa, respectively. Furthermore, from the molecular weight calculated from the ¹H-NMR of each of the both-ends amino group-terminated PBS described in Experiment Example 11 and the both-ends carboxy group-terminated PA4 described in Experiment Example 19, and the Mn of the multi-block copolymer, 1 (alphabet 'l') was calculated as 3. From the peak area ratio of the ¹H-NMR spectrum, m and n were calculated as 16 and 9, respectively. This PBS-PA4 multi-block copolymer was defined as sample 2.

### (Experiment Example 25)

A PBS-PA4 multi-block copolymer was obtained by the same methods as in Experiment Example 23, except that the both-ends amino group-terminated PBS (1.545 g, 0.50 mmol) obtained in Experiment Example 12, N,N-diisopropylethylamine (0.129 g, 1.00 mmol), N,N-dimethylformamide (13 mL), lithium chloride (1.261 g, 29.75 mmol), the both-ends carboxy group-terminated PA4 (0.556 g, 0.50 mmol) obtained in Experiment Example 18, HOAt (34 mg, 0.25 mmol), and EDCI·HCl (0.422 g, 2.20 mmol) were used. The yield amount was 2.05 g and the yield was 95%. The molecular weight distribution measurement was performed by GPC, and Mn and Mw were calculated as 28.9 kDa and 105.3 kDa, respectively. Furthermore, from the molecular weight calculated from the ¹H-NMR of each of the both-ends amino group-terminated PBS described in Experiment Example 12 and the both-ends carboxy group-terminated PA4 described in Experiment Example 18, and the Mn of the multi-block copolymer, l (alphabet 'l') was calculated as 7. From the peak area ratio of the ¹H-NMR spectrum, m and n were calculated as 17 and 5, respectively. This PBS-PA4 multi-block copolymer was defined as sample 3.

### (Experiment Example 26)

A PBS-PA4 multi-block copolymer was obtained by the same methods as in Experiment Example 23, except that the both-ends amino group-terminated PBS (1.889 g, 0.50 mmol) obtained in Experiment Example 13, N,N-diisopropylethylamine (0.129 g, 1.00 mmol), N,N-dimethylformamide (15 mL), lithium chloride (1.500 g, 35.39 mmol), the both-ends carboxy group-terminated PA4 (1.293 g, 0.50 mmol) obtained in Experiment Example 21, HOAt (136 mg, 1.00 mmol), and EDCI·HCl (0.211 g, 1.10 mmol) were used. The yield amount was 2.95 g and the yield was 94%. The molecular weight distribution measurement was performed by GPC, and Mn and Mw were calculated as 21.5 kDa and 76.3 kDa, respectively. Furthermore, from the molecular weight calculated from the ¹H-NMR of each of the both-ends amino group-terminated PBS described in Experiment Example 13 and the both-ends carboxy group-terminated PA4 described in Experiment Example 21, and the Mn of the multi-block copolymer, l (alphabet 'l') was calculated as 3. From the peak area ratio of the ¹H-NMR spectrum, m and n were calculated as 22 and 16, respectively. This PBS-PA4 multi-block copolymer was defined as sample 4.

### (Experiment Example 27)

A PBS-PA4 multi-block copolymer was obtained by the same methods as in Experiment Example 23, except that the both-ends amino group-terminated PBS (1.545 g, 0.50 mmol) obtained in Experiment Example 12, N,N-diisopropylethylamine (0.129 g, 1.00 mmol), N,N-dimethylformamide (15 mL), lithium chloride (1.500 g, 35.39 mmol), the both-ends carboxy group-terminated PA4 (1.831 g, 0.50 mmol) obtained in Experiment Example 22, HOAt (136 mg, 1.00 mmol), and EDCI·HCl (0.211 g, 1.10 mmol) were used. The yield amount was 3.08 g and the yield was 93%. The molecular weight distribution measurement was performed by GPC, and Mn and Mw were calculated as 21.7 kDa and 85.0 kDa, respectively. Furthermore, from the molecular weight calculated from the ¹H-NMR of each of the both-ends amino group-terminated PBS described in Experiment Example 12 and the both-ends carboxy group-terminated PA4 described in Experiment Example 22, and the Mn of the multi-block copolymer, l (alphabet 'l') was calculated as 3. From the peak area ratio of the ¹H-NMR spectrum, m and n were calculated as 18 and 20, respectively. This PBS-PA4 multi-block copolymer was defined as sample 5.

### (Experiment Example 28)

A PBS-PA4 multi-block copolymer was obtained by the same methods as in Experiment Example 23, except that the both-ends amino group-terminated PBS (2.835 g, 0.50 mmol) obtained in Experiment Example 14, N,N-diisopropylethylamine (0.129 g, 1.00 mmol), N,N-dimethylformamide (15 mL), lithium chloride (1.500 g, 35.39 mmol), the both-ends carboxy group-terminated PA4 (0.556 g, 0.50 mmol) obtained in Experiment Example 18, HOAt (136 mg, 1.00 mmol), and EDCI·HCl (0.211 g, 1.10 mmol) were used. The yield amount was 3.17 g and the yield was 95%. The molecular weight distribution measurement was performed by GPC, and Mn and Mw were calculated as 28.1 kDa and 75.7 kDa, respectively. Furthermore, from the molecular weight calculated from the ¹H-NMR of each of the both-ends amino group-terminated PBS described in Experiment Example 14 and the both-ends carboxy group-terminated PA4 described in Experiment Example 18, and the Mn of the multi-block copolymer, l (alphabet 'l') was calculated as 4. From the peak area ratio of the ¹H-NMR spectrum, m and n were calculated as 32 and 5, respectively. This PBS-PA4 multi-block copolymer was defined as sample 6.

### (Experiment Example 29)

A PBS-PA4 multi-block copolymer was obtained by the same methods as in Experiment Example 23, except that the both-ends amino group-terminated PBS (2.268 g, 0.40 mmol) obtained in Experiment Example 14, N,N-diisopropylethylamine (0.103 g, 0.80 mmol), N,N-dimethylformamide (15 mL), lithium chloride (1.500 g, 35.39 mmol), the both-ends carboxy group-terminated PA4 (0.819 g, 0.40 mmol) obtained in Experiment Example 20, HOAt (109 mg, 0.80 mmol), and EDCI·HCl (0.109 g, 0.88 mmol) were used. The yield amount was 2.88 g and the yield was 95%. The molecular weight distribution measurement was performed by GPC, and Mn and Mw were calculated as 33.0 kDa and 87.9 kDa, respectively. Furthermore, from the molecular weight calculated from the ¹H-NMR of each of the both-ends amino group-terminated PBS described in Experiment Example 14 and the both-ends carboxy group-terminated PA4 described in Experiment Example 20, and the Mn of the multi-block copolymer, l (alphabet 'l') was calculated as 4. From the peak area ratio of the ¹H-NMR spectrum, m and n were calculated as 34 and 10, respectively. This PBS-PA4 multi-block copolymer was defined as sample 7.

### (Experiment Example 30)

A PBS-PA4 multi-block copolymer was obtained by the same methods as in Experiment Example 23, except that the both-ends amino group-terminated PBS (2.268 g, 0.40 mmol) obtained in Experiment Example 14, N,N-diisopropylethylamine (0.103 g, 0.80 mmol), N,N-dimethylformamide (15 mL), lithium chloride (1.500 g, 35.39 mmol), the both-ends carboxy group-terminated PA4 (1.465 g, 0.40 mmol) obtained in Experiment Example 22, HOAt (109 mg, 0.80 mmol), and EDCI·HCl (0.169 g, 0.88 mmol) were used. The yield amount was 3.46 g and the yield was 94%. The molecular weight distribution measurement was performed by GPC, and Mn and Mw were calculated as 39.2 kDa and 111.0 kDa, respectively. Furthermore, from the molecular weight calculated from the ¹H-NMR of each of the both-ends amino group-terminated PBS described in Experiment Example 14 and the both-ends carboxy group-terminated PA4 described in Experiment Example 22, and the Mn of the multi-block copolymer, l (alphabet 'l') was calculated as 4. From the peak area ratio of the ¹H-NMR spectrum, m and n were calculated as 35 and 20, respectively. This PBS-PA4 multi-block copolymer was defined as sample 8.

### (Experiment Example 31)

A PBS-PA4 multi-block copolymer was obtained by the same methods as in Experiment Example 23, except that the both-ends amino group-terminated PBS (2.776 g, 0.30 mmol) obtained in Experiment Example 15, N,N-diisopropylethylamine (0.078 g, 0.60 mmol), N,N-dimethylformamide (15 mL), lithium chloride (1.500 g, 35.39 mmol), the both-ends carboxy group-terminated PA4 (0.334 g, 0.30 mmol) obtained in Experiment Example 18, HOAt (82 mg, 0.60 mmol), and EDCI·HCl (0.127 g, 0.66 mmol) were used. The yield amount was 2.99 g and the yield was 97%. The molecular weight distribution measurement was performed by GPC, and Mn and Mw were calculated as 27.6 kDa and 73.0 kDa, respectively. Furthermore, from the molecular weight calculated from the ¹H-NMR of each of the both-ends amino group-terminated PBS described in Experiment Example 15 and the both-ends carboxy group-terminated PA4 described in Experiment Example 18, and the Mn of the multi-block copolymer, l (alphabet 'l') was calculated as 3. From the peak area ratio of the ¹H-NMR spectrum, m and n were calculated as 55 and 5, respectively. This PBS-PA4 multi-block copolymer was defined as sample 9.

### (Experiment Example 32)

A PBS-PA4 multi-block copolymer was obtained by the same methods as in Experiment Example 23, except that the both-ends amino group-terminated PBS (2.776 g, 0.30 mmol) obtained in Experiment Example 15, N,N-diisopropylethylamine (0.078 g, 0.60 mmol), N,N-dimethylformamide (15 mL), lithium chloride (1.500 g, 35.39 mmol), the both-ends carboxy group-terminated PA4 (0.614 g, 0.30 mmol) obtained in Experiment Example 20, HOAt (82 mg, 0.60 mmol), and EDCI·HCl (0.127 g, 0.66 mmol) were used. The yield amount was 3.20 g and the yield was 95%. The molecular weight distribution measurement was performed by GPC, and Mn and Mw were calculated as 28.9 kDa and 80.3 kDa, respectively. Furthermore, from the molecular weight calculated from the ¹H-NMR of each of the both-ends amino group-terminated PBS described in Experiment Example 15 and the both-ends carboxy group-terminated PA4 described in Experiment Example 20, and the Mn of the multi-block copolymer, l (alphabet 'l') was calculated as 2. From the peak area ratio of the ¹H-NMR spectrum, m and n were calculated as 59 and 10, respectively. This PBS-PA4 multi-block copolymer was defined as sample 10.

### (Experiment Example 33)

A PBS-PA4 multi-block copolymer was obtained by the same methods as in Experiment Example 23, except that the both-ends amino group-terminated PBS (2.314 g, 0.25 mmol) obtained in Experiment Example 15, N,N-diisopropylethylamine (0.065 g, 0.50 mmol), N,N-dimethylformamide (15 mL), lithium chloride (1.500 g, 35.39 mmol), the both-ends carboxy group-terminated PA4 (0.916 g, 0.25 mmol) obtained in Experiment Example 22, HOAt (68 mg, 0.50 mmol), and EDCI·HCl (0.105 g, 0.55 mmol) were used. The yield amount was 2.97 g and the yield was 93%. The molecular weight distribution measurement was performed by GPC, and Mn and Mw were calculated as 29.9 kDa and 81.6 kDa, respectively. Furthermore, from the molecular weight calculated from the ¹H-NMR of each of the both-ends amino group-terminated PBS described in Experiment Example 15 and the both-ends carboxy group-terminated PA4 described in Experiment Example 22, and the Mn of the multi-block copolymer, l (alphabet 'l') was calculated as 2. From the peak area ratio of the ¹H-NMR spectrum, m and n were calculated as 53 and 17, respectively. This PBS-PA4 multi-block copolymer was defined as sample 11.

For samples 1 to 11 described above, Experiment Example numbers in each step are summarized in Table 1.

**[Table 1]**

| Step | Step1 | Step2 | Step3 | Step4 | Step5 | Step6 |
|---|---|---|---|---|---|---|
| Experiment Example No. | 1-5 | 6-10 | 11-15 | 16 | 17-22 | 23-33 |
| Sample1 | 1 | 6 | 11 | 16 | 17 | 23 |
| Sample2 | 1 | 6 | 11 | 16 | 19 | 24 |
| Sample3 | 2 | 7 | 12 | 16 | 18 | 25 |
| Sample4 | 3 | 8 | 13 | 16 | 21 | 26 |
| Sample5 | 2 | 7 | 12 | 16 | 22 | 27 |
| Sample6 | 4 | 9 | 14 | 16 | 18 | 28 |
| Sample7 | 4 | 9 | 14 | 16 | 20 | 29 |
| Sample8 | 4 | 9 | 14 | 16 | 22 | 30 |
| Sample9 | 5 | 10 | 15 | 16 | 18 | 31 |
| Sample10 | 5 | 10 | 15 | 16 | 20 | 32 |
| Sample11 | 5 | 10 | 15 | 16 | 22 | 33 |

In Experiment Examples 1 to 33 described above, the GPC was measured by the below-described method.

Using, as an eluant, 1,1,1,3,3,3-hexafluoro-2-propanol added with 0.05 M triethylamine, measurement was performed using a measurement apparatus including a degassing device (FG-32) (23.0 kPa) manufactured by FLOM Corporation, a liquid feeding pump (PU-4185) (flow rate: 0.15 mL/min) manufactured by Jasco Corporation, an autosampler (AS-4150) (20 µL sample loop, sample concentration: 1 mg/mL) manufactured by Jasco Corporation, a column oven (CO-4060) (40°C) manufactured by Jasco Corporation, an RI detector (RI-4035) manufactured by Jasco Corporation, and columns (LF-404 and LF-G) manufactured by Showa Denko K.K. The molecular weights of the samples were calculated by a calibration curve prepared from polymethyl methacrylate standard samples (molecular weight: 3040, 7290, 20100, 72000, 224000, 539000, and 1020000) manufactured by Showa Denko K.K.

In Experiment Examples 1 to 33 described above, ¹H-NMR was measured using a nuclear magnetic resonance spectrometer (JNM-ECX400) manufactured by JEOL Ltd. In Experiment Examples 1 to 16, 10 mg of a specimen was dissolved in deuterated chloroform (manufactured by Aldrich, 1 mL, deuteration rate: 99.8% or more, containing 0.03 vol% tetramethyl silane) to prepare a measurement sample. In Experiment Examples 17 to 33, 10 mg of a specimen was dissolved in deuterated trifluoroacetic acid (manufactured by Kanto Chemical Co., Inc., 1 mL, deuteration rate: 99.5%) to prepare a measurement sample.

### (Evaluation of PBS-PA4 multi-block copolymer)

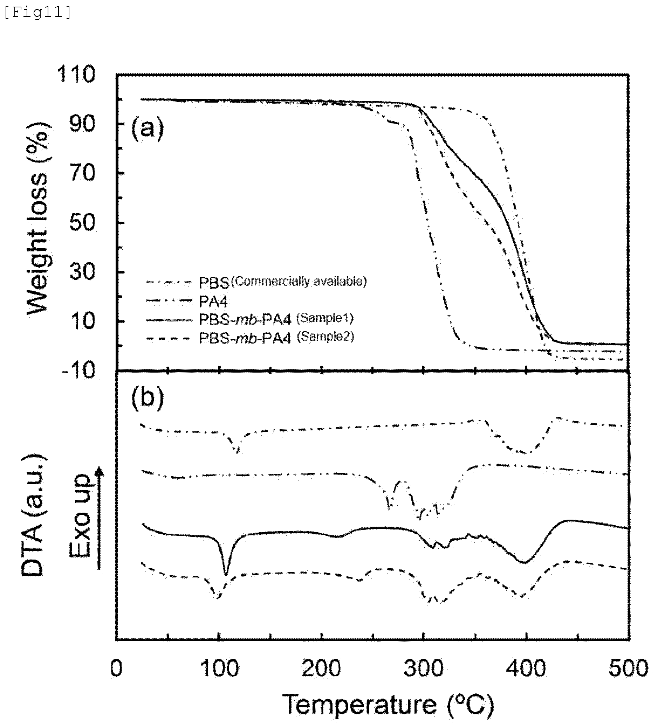

Fig. 11 shows thermal properties of PBS-PA4 multi-block copolymers (sample 1 (m = 16, n = 6, l = 3) and sample 2 (m = 16, n = 9, l = 3)). The thermal properties were evaluated by simultaneous thermogravimetric and differential thermal analysis (TG-DTA). The measurement was performed by weighing and placing about 5 mg of each sample on an aluminum pan and using DTG-60 manufactured by Shimadzu Corporation under the conditions of a temperature increase rate from room temperature to 600°C at 10°C/min under a nitrogen flow at 100 mL/min. For comparison, Fig. 11 also shows thermal properties data of PBS (manufactured by Mitsubishi Chemical Corporation, product name: Bio PBS, product number: FZ91PM) and PA4.

PA4 was prepared according to the method of NPL 1. Specifically, ring-opening polymerization was performed by the same methods as in Experiment Example 17 using, as an initiator, a condensate of benzoyl chloride and 2-pyrrolidone to synthesize PA4. The molecular weight of the prepared PA4 was calculated by GPC measurement, and Mn and Mw were calculated as 12.7 kDa and 64.6 kDa, respectively. The thermal properties of the PBS and the PA4 were measured by the same methods under the same conditions as in sample 1. Furthermore, the thermal properties of samples 3 to 11 were also measured by the same method under the same conditions as in sample 1 or the like. The thermal properties of these samples 1 to 11, PA4, and PBS are summarized in Table 2. In Table 2, "Tm1" is a first melting point derived from PBS, "Tm2" is a second melting point derived from PA4, and "Td10" is a temperature at which 10% by mass relative to the total weight of the multi-block copolymer decomposes (disappears).

**[Table 2]**

| | m | n | l | Tm1(°C) | Tm2(°C) | Td10(°C) |
|---|---|---|---|---|---|---|
| Sample1 (Experiment Example 23) | 16 | 6 | 3 | 107 | 216 | 309 |
| Sample2 (Experiment Example 24) | 16 | 9 | 3 | 99 | 237 | 303 |
| Sample3 (Experiment Example 25) | 17 | 5 | 7 | 101 | 211 | 315 |
| Sample4 (Experiment Example 26) | 22 | 16 | 3 | 100 | 248 | 301 |
| Sample5 (Experiment Example 27) | 18 | 20 | 3 | 95 | 257 | 299 |
| Sample6 (Experiment Example 28) | 32 | 5 | 4 | 111 | 201 | 312 |
| Sample7 (Experiment Example 29) | 34 | 10 | 4 | 101 | 244 | 305 |
| Sample8 (Experiment Example 30) | 35 | 20 | 4 | 98 | 260 | 308 |
| Sample9 (Experiment Example 31) | 55 | 5 | 3 | 115 | 201 | 325 |
| Sample10 (Experiment Example 32) | 59 | 10 | 2 | 113 | 225 | 307 |
| Sample11 (Experiment Example 33) | 53 | 17 | 2 | 112 | 254 | 304 |
| PBS(Commercially available) | - | - | - | 117 | - | 365 |
| PA4 | - | - | - | - | 267 | 277 |

As described above, the polyamide 4 has a melting point close to a decomposition temperature (270 to 280°C). However, it was confirmed from Fig. 11 that the PBS-PA4 multi-block copolymer (sample 1) (m = 16, n = 6) has separate melting points because PBS/PA4 are not compatible, and the melting point lowered from 117°C as the melting point of only PBS to 107°C, and from 267°C as the melting point of only PA4 to 216°C. That is, it can be said that according to the PBS-PA4 multi-block copolymer, the melting point became significantly lower than the decomposition temperature (270 to 280°C) of PA4, so that good moldability was realized. On the other hand, it was found that, since the melting point of only PA4 of the PBS-PA4 multi-block copolymer (sample 2) (m = 16, n = 9) was observed to be 237°C, heat resistance can be improved by increasing the PA4 component. Furthermore, in samples 3 to 11, heat resistance was also changed by the PA4 component. In addition, it can be said that the temperature range between "Tm2" and "Td10" becomes wider than that of PA4 itself, so that moldability is good. Furthermore, the multi-block copolymer of a polyester and a polyamide obtained is a copolymer that has better heat resistance than a homopolymer of an ester component and that is moldable at a temperature lower than a homopolymer of an amide component. In particular, "Tm2" that is the second melting point derived from PA4 can be adjusted by adjusting the value of n, and "Tm2" decreases when the value of n is small, so that molding at a lower temperature is possible. On the other hand, "Tm2" increases when the value of n is large, so that heat resistance improves. The value of n is suitably in a range of 5 to 20. When the value of n is smaller than the range, the effect of improving heat resistance is low, and it cannot be said that purification of a precursor is easy. On the other hand, when the value of n is as large as more than the above-described range, the effect of improving moldability deteriorates, and the effect of improving heat resistance is plateaued.

Next, a film was prepared from the PBS-PA4 multi-block copolymer, and the physical properties thereof were measured by the below-described method.

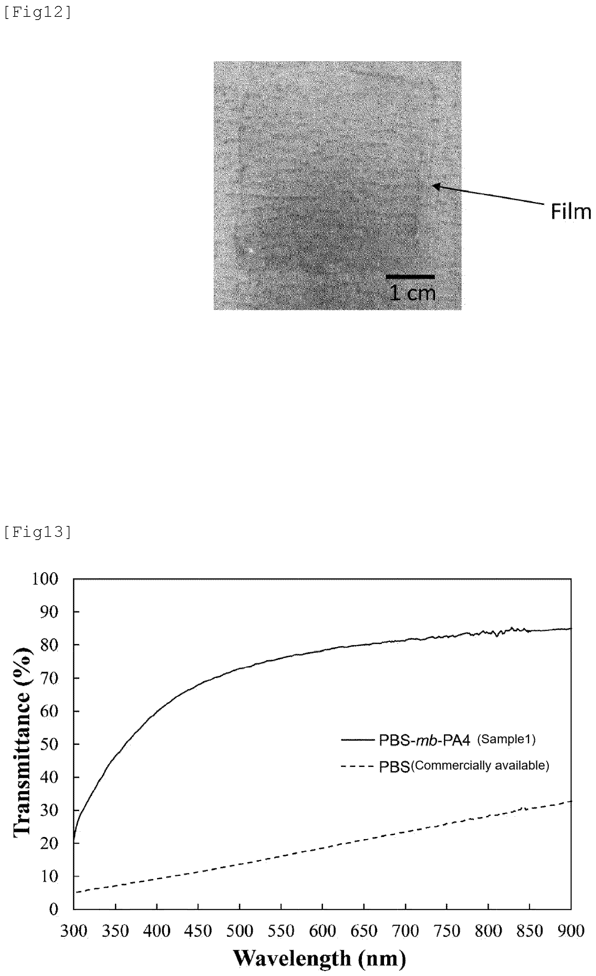

About 0.1 g of the PBS-PA4 multi-block copolymer (sample 1) obtained in Experiment Example 23 was added to about 8 mL of N,N-dimethylformamide. The mixture was stirred at 100°C and dissolved. The resultant solution was filtered through a syringe stuffed with cotton, and poured on a 4 × 4-cm glass plate edged with silicone rubber. The solvent was volatilized at 100°C to obtain a 4 × 4-cm transparent film (Fig. 12). Note that Fig. 12 is a photograph of a film placed on white paper waste. The film had a thickness of about 60 µm (micrometer). In this manner, it was confirmed that the PBS-PA4 multi-block copolymer of Example 1 can also be easily molded into a film. Then, this film was irradiated with light having a wavelength of 260 to 1600 nm at a 1-nm step using a UV-visible spectrophotometer (manufactured by Shimadzu Corporation, SoliedSpec-3700) to measure the light transmittance. Fig. 13 shows the measurement result of the light transmittance of the film. Note that Fig. 13 also shows a result of the PBS film obtained by the same methods for comparison. The PBS film was prepared using about 0.1 g of the above-described commercially available PBS by the same methods as those for the PBS-PA4 multi-block copolymer film. The prepared PBS film had a thickness of about 60 µm. As understood from Fig. 13, the PBS-PA4 multi-block copolymer film had a transmittance higher than that of the PBS film in the visible light region of 360 to 830 nm, and thus had excellent transparency. Therefore, this multi-block copolymer film is applicable for various usages such as food packages, protective films, and packaging materials.

Hereinafter, another embodiment of the present invention will be described in detail using Examples according to the above-described chemical reaction formula (21), but the present invention is not limited to this embodiment.

### <Example 2>

An ester-amide copolymer of the present example was synthesized through a reaction between a both-ends amino group-terminated PBS synthesized by the synthesis steps (steps 1A to 3A) of the both-ends amino group-terminated PBS and 4,4'-oxybisbenzoic acid (formula (20)).

### [Synthesis of both-ends amino group-terminated PBS]

### Step 1A (Synthesis of one-end NH-Boc-terminated butanediol (compound represented by the following formula (22)))

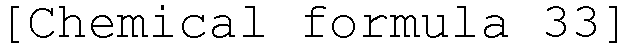

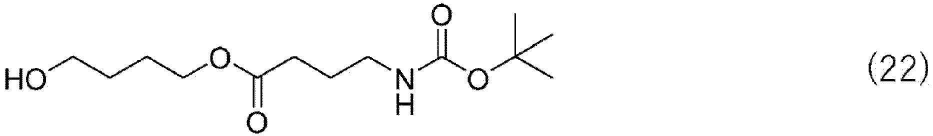

### (Experiment Example 34)

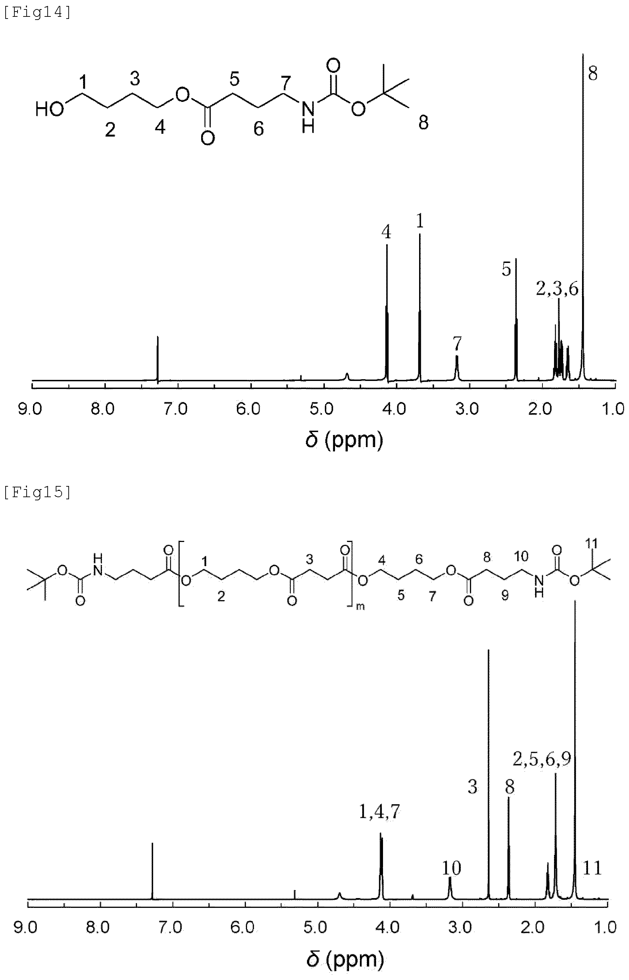

Into a 100 mL recovery flask, 1,4-butanediol (manufactured by FUJIFILM Wako Pure Chemical Corporation, 15.771 g, 175.00 mmol), 4-(tert-butoxycarbonylamino)butyric acid (manufactured by Tokyo Chemical Industry Co., Ltd., 7.113 g, 35.00 mmol), DMAP (manufactured by FUJIFILM Wako Pure Chemical Corporation, 0.855 g, 7.00 mmol), and tetrahydrofuran (manufactured by FUJIFILM Wako Pure Chemical Corporation, 53 mL) were placed. The mixture was cooled in an ice bath, added with EDCI·HCl (manufactured by Peptide Institute, Inc., 10.065 g, 52.50 mmol), and stirred at room temperature for 4 hours. The reaction solution was concentrated, and then added with 100 mL of ethyl acetate to be diluted. The diluted solution was liquid-separated and washed with 50 mL of a 1M KHSO₄ aqueous solution and 50 mL of saturated NaHCO₃ in this order. The obtained organic layer was collected, added with MgSO₄ for dehydration, concentrated, and purified by column chromatography (developing solution: gradient of ethyl acetate / hexane = 4/1 to 100/0) to obtain a target product (one-end NH-Boc-terminated butanediol) as a colorless and transparent liquid. The yield amount was 5.479 g, and the yield was 57%. Fig. 14 shows the measurement result of a ¹H-NMR spectrum. Since each peak could be assigned to an intended structure, it was confirmed that the target product was obtained.

### Step 2A (Synthesis of both-ends NH-Boc-terminated PBS (compound represented by the following formula (17)))

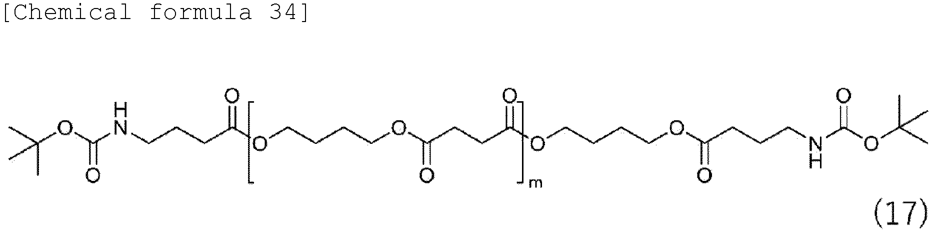

### (Experiment Example 35)

Into a 100 mL recovery flask, the one-end NH-Boc-terminated butanediol (5.000 g, 18.16 mmol) obtained in Experiment Example 34, succinic acid (manufactured by FUJIFILM Wako Pure Chemical Corporation, 1.126 g, 9.53 mmol), DMAP (manufactured by FUJIFILM Wako Pure Chemical Corporation, 0.466 g, 3.81 mmol), and dichloromethane (manufactured by FUJIFILM Wako Pure Chemical Corporation, 29 mL) were placed. The mixture was cooled in an ice bath, added with EDCI·HCl (manufactured by Peptide Institute, Inc., 5.483 g, 28.60 mmol), and stirred at room temperature for 3 hours. The reaction solution was diluted with dichloromethane to 50 mL. The diluted solution was liquid-separated and washed with 50 mL of pure water, 50 mL of a 1M KHSO₄ aqueous solution, 50 mL of saturated NaHCO₃, and 50 mL of a saturated salt solution in this order. The obtained organic layer was collected, added with MgSO₄ for dehydration, concentrated, and purified by column chromatography (developing solution: gradient of ethyl acetate / dichloromethane = 3/7 to 5/5) to obtain a target product (both-ends NH-Boc-terminated PBS) as a white solid. The yield amount was 4.128 g, and the yield was 88%. Fig. 15 shows the measurement result of a ¹H-NMR spectrum. The average degree of polymerization was calculated to be m = 1 from the peak area ratio between 10 and 3 of the ¹H-NMR spectrum shown in Fig. 15.

### Step 3A (Synthesis of both-ends amino group-terminated PBS (compound represented by the following formula (7)))

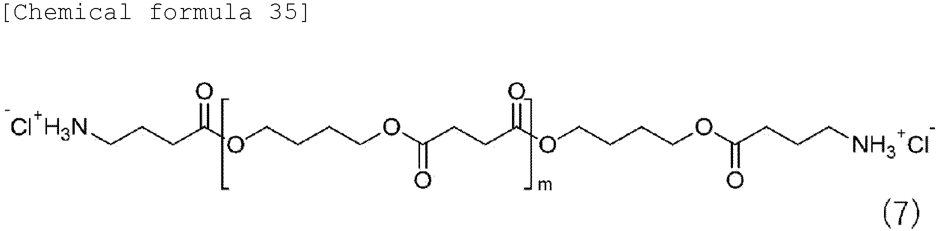

### (Experiment Example 36)

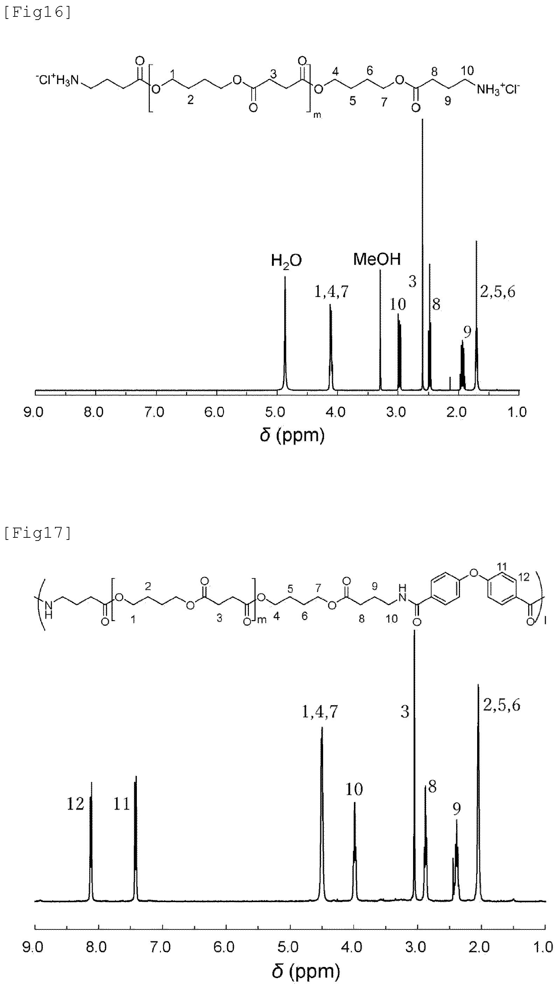

A target product (both-ends amino group-terminated PBS) was obtained as a white solid by the same method as the method according to Experiment Example 11, except that the both-ends NH-Boc-terminated PBS (4.000 g, 6.32 mmol) obtained in Experiment Example 35 and a 4 M hydrogen chloride/ethyl acetate solution (16 mL) were used. The yield amount was 3.093 g, and the yield was 94%. The average degree of polymerization was calculated to be m = 1 from the peak area ratio between 10 and 3 of the ¹H-NMR spectrum shown in Fig. 16.

### (Synthesis of ester-amide copolymer (compound represented by the following formula (19)))

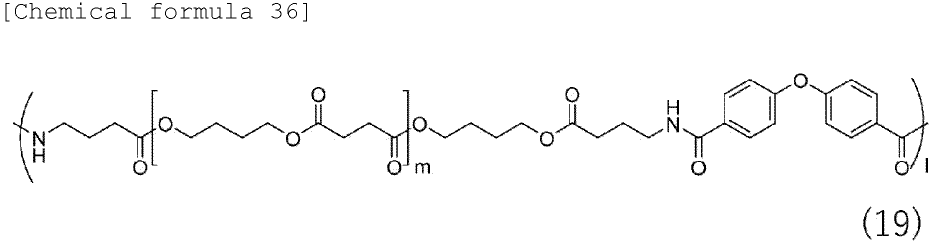

### (Experiment Example 37)

An ester-amide copolymer was obtained by the same method as in Experiment Example 23, by using the both-ends amino group-terminated PBS (0.500 g, 0.96 mmol) obtained in Experiment Example 36, N,N-diisopropylethylamine (0.249 g, 1.93 mmol), N,N-dimethylformamide (3.7 mL), HOAt (0.262 g, 1.93 mmol), and EDCI·HCl (0.406 g, 2.12 mmol) while using 4,4'-oxybisbenzoic acid (manufactured by Tokyo Chemical Industry Co., Ltd., 0.249 g, 0.963 mmol) instead of the both-ends carboxy group-terminated PA4, and performing a reaction without adding lithium chloride. The yield amount was 0.57 g, and the yield was 88%. The molecular weight was calculated by GPC to be Mn = 17.3 kDa and Mw = 132.8 kDa. Since the average degree of polymerization was calculated to be m = 1 from the peak area ratio between 11 and 3 of the ¹H-NMR spectrum shown in Fig. 17, and other peaks could be all assigned, it was confirmed that a compound having an intended structure represented by the formula (19) was obtained.

Furthermore, l (alphabet 'l') was calculated as 26 from 656 Da that is the molecular weight of repeating units. This ester-amide copolymer was defined as sample 12.

In the above-described Experiment Examples 34 to 37, ¹H-NMR was measured using a nuclear magnetic resonance spectrometer (JNM-ECX400) manufactured by JEOL Ltd. In Experiment Examples 34 to 35, 10 mg of a specimen was dissolved in deuterated chloroform (manufactured by Aldrich, 1 mL, deuteration rate: 99.8% or more, containing 0.03 vol% tetramethyl silane) to prepare a measurement sample. In Experiment Example 36, 10 mg of a specimen was dissolved in deuterated methanol (manufactured by Aldrich, 1 mL, deuteration rate: 99.8% or more) to prepare a measurement sample. In Experiment Example 37, 10 mg of a specimen was dissolved in deuterated trifluoroacetic acid (manufactured by Kanto Chemical Co., Inc., 1 mL, deuteration rate: 99.5%) to prepare a measurement sample.

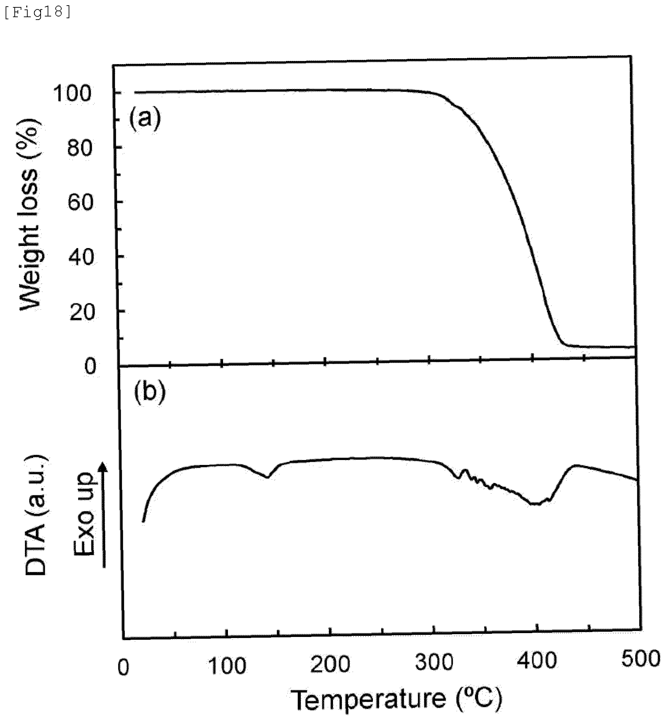

The thermal properties of sample 12 was measured by the same method under the same conditions as in sample 1. Fig. 18 and Table 3 shows the thermal properties of sample 12. Note that "Td10" is a temperature at which 10% by mass to the total weight of the ester-amide copolymer decomposes (disappears).

**[Table 3]**

| | m | n | l | Tm(°C) | Td10(°C) |
|---|---|---|---|---|---|
| Sample12 (Experiment Example 37) | 1 | - | 26 | 142 | 340 |

As shown in Fig. 18, the compound (sample 12) obtained in Experiment Example 37 exhibits a single melting point (Tm) derived from the repeating structure of an ester amide. It was found that the melting point of the resultant product was higher than that of a commercially available PBS and heat resistance had improved. Since this melting point is lower than the above-described decomposition temperature (270 to 280°C) of PA4, it can be said that moldability had improved. Furthermore, it can be said that a film can also be easily molded in the present example similarly to in Example 1. In addition, according to the present example, the heat resistance is lower than that of the multi-block copolymer of Example 1, but the moldability improves. Therefore, the present example is suitable for a packaging film for food or the like.

### Industrial Applicability

The ester-amide copolymer such as the multi-block copolymer according to the present invention is usable as films or sheets used for a food package, a protective film, and a packaging material, and other various molded articles.

## Claims

1. An ester-amide multi-block copolymer that is a multi-block copolymer including: a polyester-containing block and a polyamide-containing block obtained through ring-opening polymerization of a cyclic lactam, and that is represented by the following formula (1): 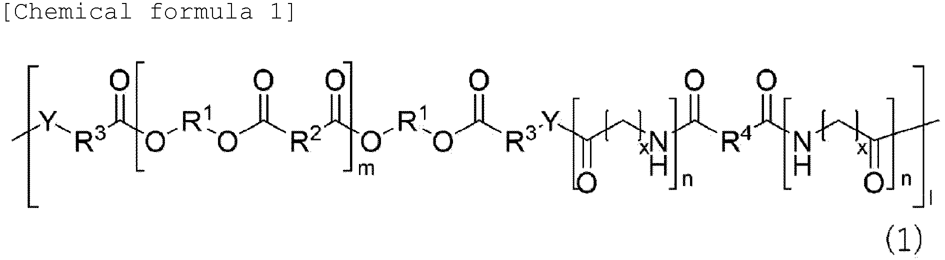 (in the formula (1), R¹ and R² each represent an alkyl chain of 1 to 20 carbon atoms having hydrogen atoms or a substituent, and R³ represents an alkyl chain of 1 to 10 carbon atoms having hydrogen atoms or a substituent; R¹, R², and R³ may be the same as or different from one another; R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 12 carbon atoms containing no hetero atoms, and an aliphatic hydrocarbon chain of 1 to 10 carbon atoms containing no hetero atoms; Y is NH, O (oxygen), or S (sulfur); m represents an integer of 1 to 60, n represents an integer of 1 to 120, 1 represents an integer of 2 to 100, and x represents an integer of 1 to 11).

2. An ester-amide multi-block copolymer that is a multi-block copolymer including a polyester-containing block and a polyamide-containing block obtained through ring-opening polymerization of a cyclic lactam, and that is represented by the following formula (2): 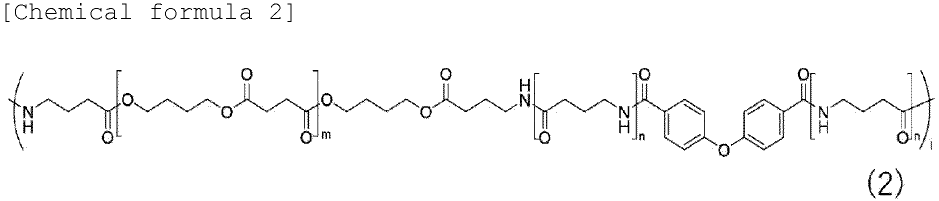 (in the formula (2), m represents an integer of 1 to 60, n represents an integer of 1 to 120, and l represents an integer of 2 to 100).

3. A film comprising the ester-amide multi-block copolymer according to claim 1 or 2.

4. A method for producing the ester-amide multi-block copolymer according to claim 1, comprising a step of bonding a polyester-containing compound represented by the following formula (3): 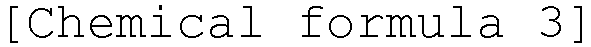 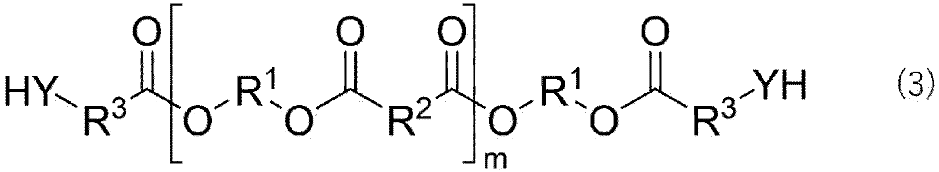 (in the formula (3), R¹ and R² each represent an alkyl chain of 1 to 20 carbon atoms having hydrogen atoms or a substituent, and R³ represents an alkyl chain of 1 to 10 carbon atoms having hydrogen atoms or a substituent; R¹, R², and R³ may be the same as or different from one another; Y is NH, O (oxygen), or S (sulfur); and m represents an integer of 1 to 60) to
a polyamide-containing compound represented by the following formula (4) obtained through ring-opening polymerization of a cyclic lactam: 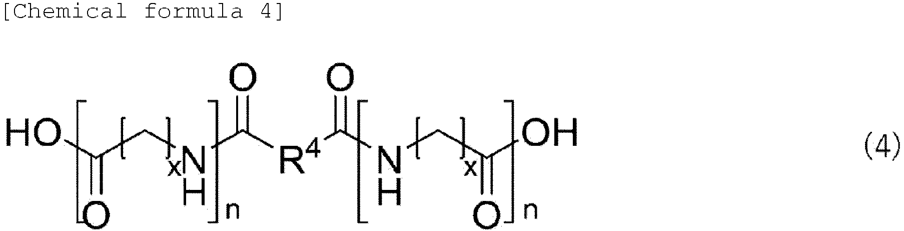 (in the formula (4), R⁴ represents any one of an aromatic chain of a heteroaromatic ring of 4 to 5 carbon atoms, a group containing two aromatic hydrocarbon chains of 6 to 12 carbon atoms and a hetero atom in between, an aliphatic hydrocarbon chain of 2 to 20 carbon atoms containing a hetero atom, an aromatic hydrocarbon chain of 6 to 12 carbon atoms containing no hetero atoms, and an aliphatic hydrocarbon chain of 1 to 10 carbon atoms containing no hetero atoms; and n represents an integer of 1 to 120, and x represents an integer of 1 to 11)
by forming a bond between a YH group in the compound represented by the formula (3) and a carboxy group in the compound represented by the formula (4).

5. A method for producing the ester-amide multi-block copolymer according to claim 2, comprising a step of bonding a polyester-containing compound represented by the following formula (5): 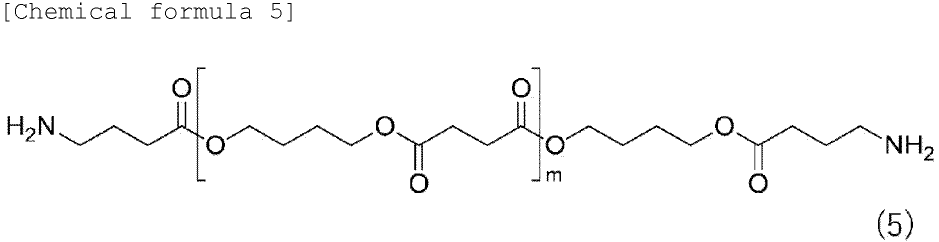 (in the formula (5), m represents an integer of 1 to 60) to
a polyamide-containing compound represented by the following formula (6) obtained through ring-opening polymerization of a cyclic lactam: 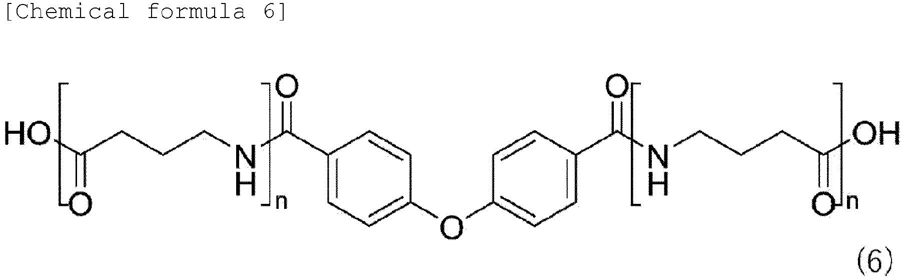 (in the formula (6), n represents an integer of 1 to 120)
by forming a bond between an amino group in the compound represented by the formula (5) and a carboxy group in the compound represented by the formula (6).

6. An ester-amide copolymer represented by the following formula (19): 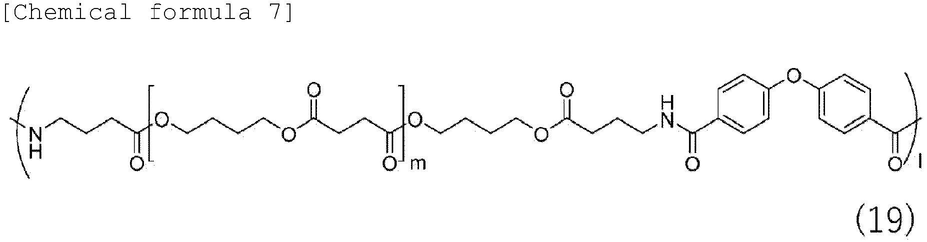 (in the formula (19), m represents an integer of 1 to 60, and l represents an integer of 2 to 100).

7. A film comprising the ester-amide copolymer according to claim 6.

8. A method for producing the ester-amide copolymer according to claim 6, comprising a step of bonding a compound represented by the following formula (5): 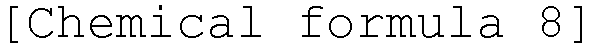 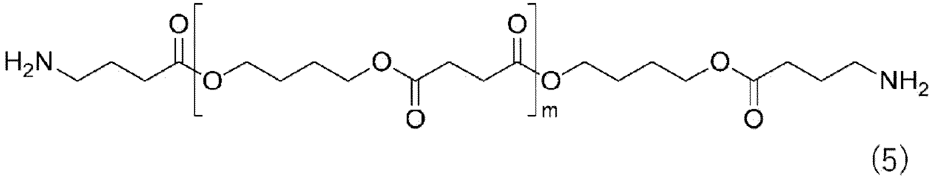 (in the formula (5), m represents an integer of 1 to 60) to
a compound represented by the following formula (20): 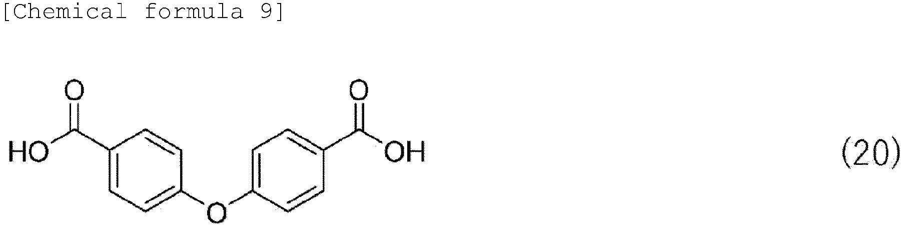
by forming a bond between an amino group in the compound represented by the formula (5) and a carboxy group in the compound represented by the formula (20).
